# EUROPEAN PATENT APPLICATION

(11) **EP 2 560 220 A1**
(43) Date of publication of application: **20.02.2013**
(21) Application number: 11768620.4
(22) Date of filing: 12.04.2011
(51) Int. Cl.: H01L 51/50, C09K 11/06

(54) **ORGANIC ELECTROLUMINESCENT ELEMENT**

(30) Priority: 12.04.2010 JP 2010091644
(71) Applicant: Idemitsu Kosan Co., Ltd., Chiyoda-ku Tokyo 100-8321 (JP)
(72) Inventor: KAWAMURA, Masahiro, Sodegaura-sh, Chiba 299-0293 (JP); KAWAMURA, Yuichiro, Sodegaura-shi, Chiba 299-0293 (JP)
(74) Representative: Gille Hrabal
(86) International application number: PCT/JP2011/002142
(87) International publication number: WO 2011/129096

(57) **Abstract**

An organic electroluminescence device sequentially includes an anode, a first organic layer, a second organic layer, and a cathode, the first organic layer including a monoanthracene derivative shown by a formula (1) and a fused aromatic amine derivative shown by a formula (2), and the second organic layer including a heterocyclic derivative shown by a formula (3).

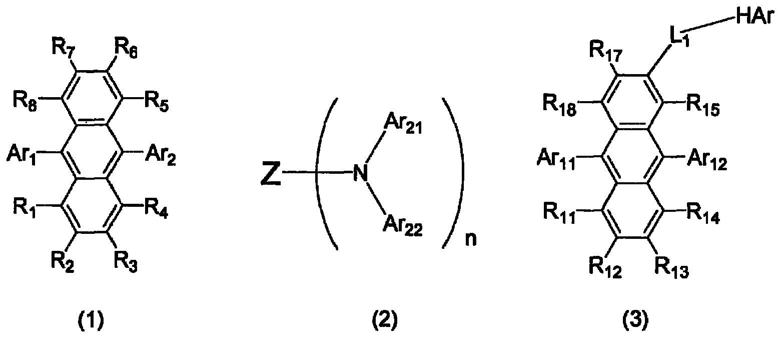

## Description

### TECHNICAL FIELD

The invention relates to an organic electroluminescence device.

### BACKGROUND ART

An organic electroluminescence (EL) device that utilizes an organic substance is a promising inexpensive solid-state emitting large full-color display, and has been extensively developed.
The organic EL device normally includes an emitting layer and a pair of opposing electrodes disposed on either side of the emitting layer. When an electric field is applied between the electrodes, electrons and holes are injected into the emitting layer respectively from the cathode and the anode. The electrons and the holes recombine in the emitting layer to produce an excited state, and the energy is emitted as light when the excited state returns to the ground state.

It is difficult to improve the performance of an organic EL device in which the hole-injecting function, the electron-injecting function, and the emitting function are implemented by a single layer. Therefore, the performance of an organic EL device has been improved by providing a plurality of organic layers that differ in function between the electrodes. A structure in which three or more layers such as a hole-transporting layer, an emitting layer, and an electron-transporting layer are stacked between two electrodes has been generally employed.

An organic EL device that was developed in an early stage was insufficient in terms of the drive voltage, the luminous efficiency, and the durability. Therefore, various technical improvements have been made to address this problem. For example, Patent Documents 1 to 3 implement a decrease in drive voltage by improving the electron-transporting material. However, the lifetime decreases when decreasing the drive voltage by improving the electron-injecting capability. An improvement in durability and a decrease in drive voltage cannot be implemented at the same time when using the technologies disclosed in Patent Documents 1 to 3.

A full-color organic EL display has been desired. It is necessary to improve the performance of a blue organic EL device, a green organic EL device, and a red organic EL device in order to implement a full-color organic EL display. However, it has been difficult to improve the performance (particularly durability) of a blue organic EL device.

### RELATED-ART DOCUMENT

### PATENT DOCUMENT

Patent Document 1: WO03/60956
Patent Document 2: WO04/80975
Patent Document 3: WO05/97756

### SUMMARY OF THE INVENTION

An object of the invention is to provide an organic EL device that can be driven at a low drive voltage and has a long lifetime.

The invention provides the following organic EL device.
1. An organic electroluminescence device sequentially including an anode, a first organic layer, a second organic layer, and a cathode, the first organic layer including a monoanthracene derivative shown by a formula (1) and a fused aromatic amine derivative shown by a formula (2), and the second organic layer including a heterocyclic derivative shown by a formula (3), 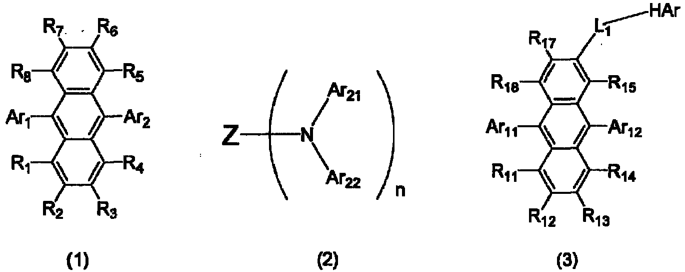 wherein R₁ to R₈, R₁₁ to R₁₅, R₁₇, and R₁₈ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 carbon atoms that form a ring (hereinafter referred to as "ring carbon atoms"), a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 atoms that form a ring (hereinafter referred to as "ring atoms"), Ar₁, Ar₂, Ar₁₁, Ar₁₂, Ar₂₁, and Ar₂₂ are independently a substituted or unsubstituted aromatic hydrocarbon ring group having 6 to 30 ring carbon atoms, or a substituted or unsubstituted heterocyclic group having 5 to 30 ring atoms, provided that the substituted aromatic hydrocarbon ring group or the substituted heterocyclic group represented by Ar₁ and Ar₂ is substituted with a substituent other than a styryl group, Z is a substituted or unsubstituted chrysene residue or a substituted or unsubstituted pyrene residue, n is an integer from 1 to 4, L₁ is a substituted or unsubstituted divalent aromatic hydrocarbon ring group having 6 to 30 ring carbon atoms, or a substituted or unsubstituted divalent heterocyclic group having 5 to 30 ring atoms, and HAr is any of nitrogen-containing heterocyclic groups shown by formulas (4) to (6), 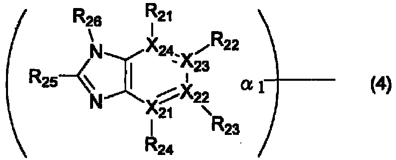 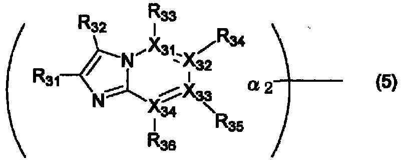 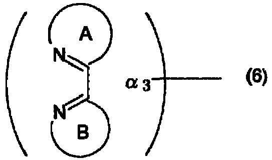 wherein R₂₁ to R₂₆ and R₃₁ to R₃₆ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituents among R₂₁ to R₂₄ and R₃₁ to R₃₆ may form a saturated or unsaturated ring, X₂₁ to X₂₄ and X₃₁ to X₃₄ are independently a carbon atom or a nitrogen atom, provided that R₂₁ is absent when X₂₄ is a nitrogen atom, R₂₂ is absent when X₂₃ is a nitrogen atom, R₂₃ is absent when X₂₂ is a nitrogen atom, and R₂₄ is absent when X₂₁ is a nitrogen atom, R₃₃ is absent when X₃₁ is a nitrogen atom, R₃₄ is absent when X₃₂ is a nitrogen atom, R₃₅ is absent when X₃₃ is a nitrogen atom, and R₃₆ is absent when X₃₄ is a nitrogen atom, A and B are independently a substituted or unsubstituted 5-membered ring or a substituted or unsubstituted 6-membered ring, provided that adjacent substituents that substitute the 5-membered ring or the 6-membered ring represented by A and B may form a saturated or unsaturated ring, α₁ is bonded to R₂₁, R₂₂, R₂₃, R₂₄, or R₂₆, α₂ is bonded to one of R₃₁ to R₃₆, and α₃ is bonded to the nitrogen-containing ring represented by A or the nitrogen-containing ring represented by B, provided that R₂₁, R₂₂, R₂₃, R₂₄, or R₂₆ that is bonded to α₁ is a single bond, and one of R₃₁ to R₃₆ that is bonded to α₂ is a single bond.
2. The organic electroluminescent device according to 1, wherein HAr is any of heterocyclic groups shown by formulas (7) to (11), 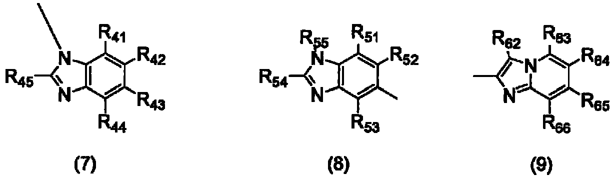 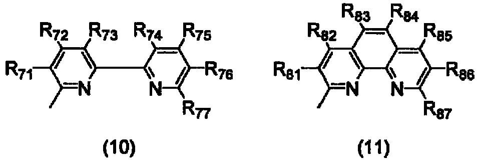 wherein R₄₁ to R₄₅, R₅₁ to R₅₅, R₆₂ to R₆₆, R₇₁ to R₇₇, and R₈₁ to R₈₇ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituents among R₄₁ to R₄₄, R₅₁, R₅₂, R₆₂ to R₆₆, R₇₁ to R₇₇, and R₈₁ to R₈₇ may bond to each other to form a saturated or unsaturated ring.
3. The organic electroluminescence device according to 1 or 2, further including a layer between the second organic layer and the cathode, the layer being formed of one or more substances selected from the group consisting of alkali metal oxides, alkali metal halides, alkaline-earth metal oxides, alkaline-earth metal halides, rare earth metal oxides, rare earth metal halides, organic complexes of alkali metals, organic complexes of alkaline-earth metals, and organic complexes of rare earth metals.
4. The organic electroluminescent device according to any one of 1 to 3, wherein the fused aromatic amine derivative shown by the formula (2) is a compound shown by a formula (12), 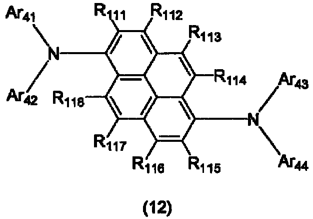 wherein R₁₁₁ to R₁₁₈ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₄₁ to Ar₄₄ are independently a substituted or unsubstituted aromatic hydrocarbon ring group having 6 to 30 ring carbon atoms or a substituted or unsubstituted heterocyclic group having 5 to 30 ring atoms.
5. The organic electroluminescence device according to 4, wherein at least one of R₁₁₂ and R₁₁₆ in the formula (12) is a substituted or unsubstituted alkyl group having 1 to 10 carbon atoms, or a substituted or unsubstituted cycloalkyl group having 3 to 10 carbon atoms.
6. The organic electroluminescence device according to any one of 1 to 3, wherein the fused aromatic amine derivative shown by the formula (2) is a compound shown by a formula (13), 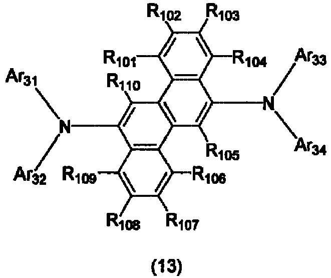 wherein R₁₀₁ to R₁₁₀ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₃₁ to Ar₃₄ are independently a substituted or unsubstituted aromatic hydrocarbon ring group having 6 to 30 ring carbon atoms or a substituted or unsubstituted heterocyclic group having 5 to 30 ring atoms.
7. The organic electroluminescence device according to any one of 1 to 6, wherein the first organic layer is adjacent to the second organic layer.

The invention thus provides an organic EL device that can be driven at a low voltage and has a long lifetime.

### DESCRIPTION OF THE EMBODIMENTS

An organic EL device according to the invention sequentially includes an anode, a first organic layer, a second organic layer, and a cathode, the first organic layer including a monoanthracene derivative shown by the following formula (1) and a fused aromatic amine derivative shown by the following formula (2), and the second organic layer including a heterocyclic derivative shown by the following formula (3). 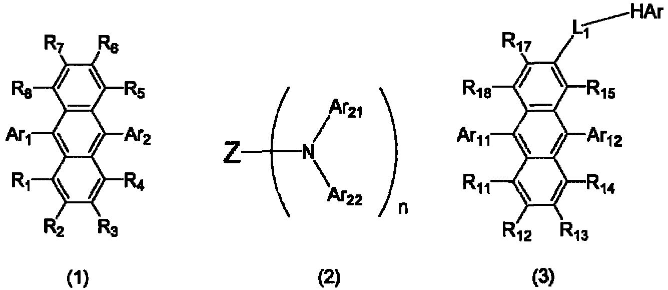 wherein R₁ to R₈, R₁₁ to R₁₅, R₁₇, and R₁₈ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, Ar₁, Ar₂, Ar₁₁, Ar₁₂, Ar₂₁, and Ar₂₂ are independently a substituted or unsubstituted aromatic hydrocarbon ring group having 6 to 30 ring carbon atoms, or a substituted or unsubstituted heterocyclic group having 5 to 30 ring atoms, provided that the substituted aromatic hydrocarbon ring group or the substituted heterocyclic group represented by Ar₁ and Ar₂ is substituted with a substituent other than a styryl group, Z is a substituted or unsubstituted chrysene residue or a substituted or unsubstituted pyrene residue, n is an integer from 1 to 4, L₁ is a substituted or unsubstituted divalent aromatic hydrocarbon ring group having 6 to 30 ring carbon atoms, or a substituted or unsubstituted divalent heterocyclic group having 5 to 30 ring atoms, and HAr is any of nitrogen-containing heterocyclic groups shown by formulas (4) to (6), 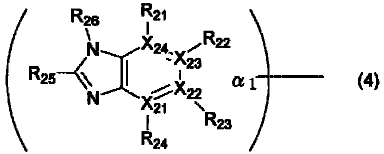 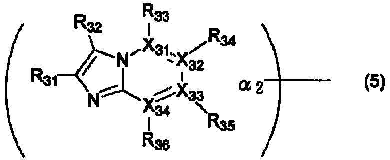 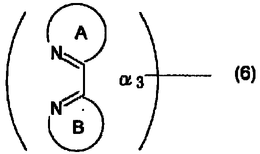 wherein R₂₁ to R₂₆ and R₃₁ to R₃₆ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituents among R₂₁ to R₂₄ and R₃₁ to R₃₆ may form a saturated or unsaturated ring, X₂₁ to X₂₄ and X₃₁ to X₃₄ are independently a carbon atom or a nitrogen atom, provided that R₂₁ is absent when X₂₄ is a nitrogen atom, R₂₂ is absent when X₂₃ is a nitrogen atom, R₂₃ is absent when X₂₂ is a nitrogen atom, R₂₄ is absent when X₂₁ is a nitrogen atom, R₃₃ is absent when X₃₁ is a nitrogen atom, R₃₄ is absent when X₃₂ is a nitrogen atom, R₃₅ is absent when X₃₃ is a nitrogen atom, and R₃₈ is absent when X₃₄ is a nitrogen atom, A and B are independently a substituted or unsubstituted 5-membered ring or a substituted or unsubstituted 6-membered ring, provided that adjacent substituents that substitute the 5-membered ring or the 6-membered ring represented by A and B may form a saturated or unsaturated ring, α₁ is bonded to R₂₁, R₂₂, R₂₃, R₂₄, or R₂₈, α₂ is bonded to one of R₃₁ to R₃₆, and α₃ is bonded to the nitrogen-containing ring represented by A or the nitrogen-containing ring represented by B, provided that R₂₁, R₂₂, R₂₃, R₂₄, or R₂₆ that is bonded to α₁ is a single bond, and one of R₃₁ to R₃₆ that is bonded to α₂ is a single bond.

### Second organic layer

In the organic EL device according to the invention, the second organic layer that includes the heterocyclic derivative shown by the formula (3) is provided between the first organic layer and the cathode, and may function as an electron-transporting layer, for example.
It is preferable that R₁₁ to R₁₈ in the heterocyclic derivative shown by the formula (3) be independently a hydrogen atom, a fluorine atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms. It is more preferable that R₁₁ to R₁₈ be independently a hydrogen atom, a substituted or unsubstituted alkyl group having 1 to 10 carbon atoms, a substituted or unsubstituted cycloalkyl group having 3 to 10 carbon atoms, a substituted or unsubstituted aromatic hydrocarbon ring group having 6 to 30 ring carbon atoms, or a substituted or unsubstituted heterocyclic group having 5 to 30 ring atoms. It is still more preferable that R₁₁ to R₁₈ be independently a hydrogen atom, a substituted or unsubstituted aromatic hydrocarbon ring group having 6 to 30 ring carbon atoms, or a substituted or unsubstituted heterocyclic group having 5 to 30 ring atoms. It is most preferable that R₁₁ to R₁₈ be hydrogen atoms.

It is preferable that Ar₁₁ and Ar₁₂ in the heterocyclic derivative shown by the formula (3) be independently a substituted or unsubstituted aromatic hydrocarbon ring group having 6 to 30 ring carbon atoms. It is more preferable that Ar₁₁ and Ar₁₂ be independently a substituted or unsubstituted aromatic hydrocarbon ring group having 6 to 20 ring carbon atoms. It is still more preferable that Ar₁₁ and Ar₁₂ be independently a substituted or unsubstituted aromatic hydrocarbon ring group having 6 to 15 ring carbon atoms. It is most preferable that Ar₁₁ and Ar₁₂ be independently a phenyl group, a naphthyl group, a phenanthryl group, a biphenyl group, or a fluorenyl group.

It is preferable that L₁ in the heterocyclic derivative shown by the formula (3) be a substituted or unsubstituted divalent aromatic hydrocarbon ring group having 6 to 30 ring carbon atoms. It is more preferable that L₁ be a substituted or unsubstituted divalent aromatic hydrocarbon ring group having 6 to 20 ring carbon atoms. It is still more preferable that L₁ be a substituted or unsubstituted divalent aromatic hydrocarbon ring group having 6 to 12 ring carbon atoms. It is most preferable that L₁ be a phenylene group.

In the nitrogen-containing heterocyclic group represented by HAr in the heterocyclic derivative shown by the formula (3) that is included in the second organic layer, a large substituent (e.g., anthracene) is not present around C=N (i.e., electron-injecting site). Therefore, acceptance of electrons is rarely hindered by steric hindrance, so that the electron-injecting capability of the second organic layer is improved. It is conjectured that the organic EL device can be driven at a low drive voltage for the above reason.

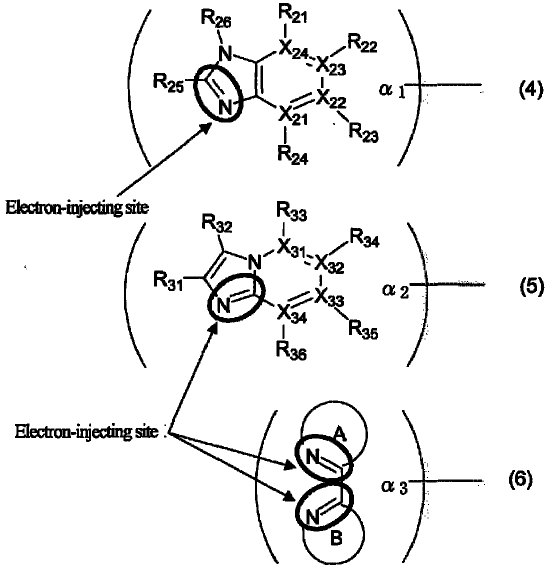

The nitrogen-containing heterocyclic group represented by HAr enhances interaction with a metal that forms the cathode, or an alkali metal salt or an organic metal complex that is present at the interface between the cathode and an electron-transporting material. It is conjectured that the electron-injecting capability increases as the interaction increases, so that the drive voltage of the organic EL device can be reduced.
It is effective to introduce a structure that increases the coordination capability with a metal in order to increase the interaction. Any of the nitrogen-containing heterocyclic groups shown by the formulas (4) and (5) that have an imidazole structure and the nitrogen-containing heterocyclic group shown by the formula (6) is used as HAr taking account of the electron resistance of the heterocyclic ring.

R₂₅ in the formula (4) is preferably an aromatic hydrocarbon ring group having 6 to 12 ring carbon atoms or an alkyl group having 1 to 10 carbon atoms.
A charge tends to be delocalized at a 2-position substituent of benzimidazole as compared with a 1-position substituent. Therefore, a charge is easily delocalized when the 2-position substituent has a large number of ring carbon atoms, so that the electron density of the nitrogen atom of benzimidazole decreases, and interaction with a metal becomes weak. This may result in a decrease in electron-injecting capability. Therefore, it is preferable that the 2-position substituent of benzimidazole be a relatively small substituent such as an aromatic hydrocarbon ring group having 6 to 12 ring carbon atoms or an alkyl group having 1 to 10 carbon atoms in order to prevent charge delocalization.

Heterocyclic groups shown by the following formulas (7) to (9) that have a benzimidazole structure or an imidazopyridine structure are preferable as the nitrogen-containing heterocyclic groups shown by the formula (4) and (5) that have an imidazole structure.
A group that has a bipyridyl structure, a phenanthrolyl structure, a pyridylpyrimidyl structure, or a pyridyltriadinyl structure is preferable as the nitrogen-containing heterocyclic group shown by the formula (6). Heterocyclic groups shown by the following formulas (10) and (11) are more preferable as the nitrogen-containing heterocyclic group shown by the formula (6). 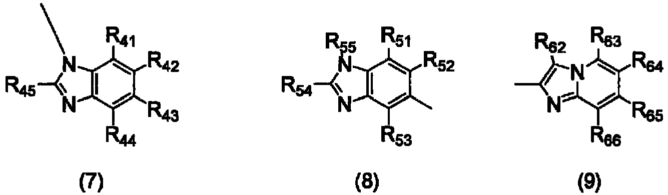 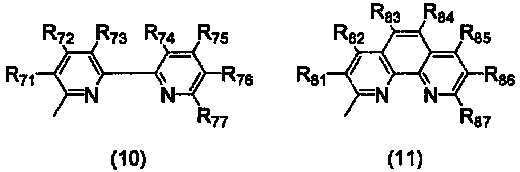 wherein R₄₁ to R₄₅, R₅₁ to R₅₅, R₆₂ to R₆₆, R₇₁ to R₇₇, and R₈₁ to R₈₇ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituent among R₄₁ to R₄₄, R₅₁, R₅₂, R₆₂ to R₆₆, R₇₁ to R₇₇, and R₈₁ to R₈₇ may bond to each other to form a saturated or unsaturated ring.

Adjacent substituent among R₂₁ to R₂₄, R₃₁ to R₃₆, R₄₁ to R₄₅, R₅₁ to R₅₅, R₆₂ to R₆₆, R₇₁ to R₇₇, and R₈₁ to R₈₇ in the formulas (4) to (11) may bond to each other to form a saturated or unsaturated ring. Specific examples of the heterocyclic derivative shown by the formula (3) in which adjacent substituents bond to each other to form a ring include the compounds shown by the formulas ET2-34 to ET2-38, ET2-41 to ET2-45, and ET2-48.

R₄₅ in the heterocyclic group shown by the formula (7) is preferably an aromatic hydrocarbon ring group having 6 to 12 ring carbon atoms or an alkyl group having 1 to 10 carbon atoms, and more preferably an alkyl group having 1 to 10 carbon atoms. The drive voltage of the organic EL device can be reduced when HAr is the heterocyclic group shown by the formula (7), and R₄₅ is an alkyl group.

Examples of the alkyl group having 1 to 10 carbon atoms represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, and R₈₁ to R₈₇ in the heterocyclic derivative included in the second organic layer include an ethyl group, a methyl group, an i-propyl group, an n-propyl group, an s-butyl group, a t-butyl group, a pentyl group, a hexyl group, and the like. The alkyl group is preferably an alkyl group having 1 to 8 carbon atoms, and more preferably an alkyl group having 1 to 6 carbon atoms.

Examples of the halogen atom represented by R₁₁ to R₁₅, R₁₇, R₁₈, R₂₁ to R₂₆, R₃₁ to R₃₈, R₄₁ to R₄₅, R₅₁ to R₅₅, R₆₂ to R₆₆, R₇₁ to R₇₇, and R₈₁ to R₈₇ include a fluorine atom, a chlorine atom, a bromine atom, an iodine atom, and the like. Among these, a fluorine atom is preferable.

Examples of the cycloalkyl group having 3 to 10 carbon atoms represented by R₁₁ to R₁₅, R₁₇, R₁₈, R₂₁ to R₂₆, R₃₁ to R₃₆, R₄₁ to R₄₅, R₅₁, to R₅₅, R₆₂ to R₆₆, R₇₁ to R₇₇, and R₈₁ to R₈₇ include a cyclopropyl group, a cyclobutyl group, a cyclopentyl group, a cyclohexyl group, a 4-methylcyclohexyl group, and the like. The cycloalkyl group is preferably a cycloalkyl group having 3 to 6 carbon atoms.

Examples of the alkylsilyl group having 3 to 20 carbon atoms represented by R₁₁ to R₁₅, R₁₇, R₁₈, R₂₁ to R₂₆, R₃₁ to R₃₆, R₄₁ to R₄₅, R₅₁, to R₅₅, R₆₂ to R₆₆, R₇₁ to R₇₇, and R₈₁ to R₈₇ include a trimethylsilyl group, a triethylsilyl group, a t-butyidimethylsilyl group, a vinyldimethylsilyl group, a propyldimethylsilyl group, and the like. The alkylsilyl group is preferably an alkylsilyl group having 3 to 10 carbon atoms.

The arylsilyl group having 6 to 30 ring carbon atoms (or 8 to 30 ring carbon atoms) represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, and R₈₁ to R₈₇ is a group obtained by substituting a silyl group with an aromatic hydrocarbon ring group. The silyl group may also be substituted with a group other than the aromatic hydrocarbon ring group. Specifically, the term "arylsilyl group" used herein includes an arylalkylsilyl group and the like. Examples of the arylsilyl group include a triphenylsilyl group, a phenyldimethylsilyl group, a t-butyldiphenylsilyl group, a tritolylsilyl group, a trixylylsilyl group, a trinaphthylsilyl group, and the like. The arylsilyl group is preferably an arylsilyl group having 6 to 20 ring carbon atoms, and more preferably an arylsilyl group having 6 to 12 ring carbon atoms.

The alkoxy group having 1 to 10 carbon atoms represented by R₁₁ to R₁₅, R₁₇, R₁₈, R₂₁ to R₂₆, R₃₁ to R₃₆, R₄₁ to R₄₅, R₅₁ to R₅₅, P₆₂ to R₆₆, R₇₁ to R₇₇, and R₈₁ to R₈₇ is a group shown by -OY. Examples of Y include those mentioned above in connection with the alkyl group. The alkoxy group is preferably an alkoxy group having 1 to 8 carbon atoms, and more preferably an alkoxy group having 1 to 6 carbon atoms.

The aryloxy group having 6 to 30 carbon atoms represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, and R₈₁ to R₈₇ is a group shown by -OAr. Examples of Ar include those mentioned below in connection with an aromatic hydrocarbon ring group. The aryloxy group is preferably an aryloxy group having 6 to 20 ring carbon atoms, and more preferably an aryloxy group having 6 to 12 ring carbon atoms.

Examples of the aromatic hydrocarbon ring group having 6 to 30 ring carbon atoms represented by R₁₁ to R₁₅, R₁₇, R₁₈, R₂₁ to R₂₆, R₃₁ to R₃₈, R₄₁ to R₄₅, R₅₁ to R₅₅, R₆₂ to R₆₆, R₇₁ to R₇₇, R₈₁ to R₈₇, Ar₁₁, and Ar₁₂ include a phenyl group, a naphthyl group, a phenanthryl group, a biphenyl group, a terphenyl group, an anthryl group, a chrysenyl group, a benzophenanthryl group, a benzanthryl group, a benzochrysenyl group, a fluorenyl group, a fluoranthenyl group, a naphthaoenyl group, a pentacenyl group, a perylenyl group, a picenyl group, a pyrenyl group, a pentaphenylenyl group, and the like. The aromatic hydrocarbon ring group is preferably an aromatic hydrocarbon ring group having 6 to 20 ring carbon atoms, and more preferably an aromatic hydrocarbon ring group having 6 to 15 ring carbon atoms.
Note that the term "aromatic hydrocarbon ring group" used herein refers to a substituent that includes an aromatic monocyclic ring, an aromatic fused ring, or a plurality of aromatic monocyclic rings and/or aromatic fused rings that are bonded via a single bond, and excludes a substituent that includes a double bond and does not include only a cyclic structure (e.g., styryl group).

Examples of the heterocyclic group having 5 to 30 ring atoms represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, R₈₁ to R₈₇, Ar₁₁, and Ar₁₂ include a pyridinyl group, a pyrazinyl group, a pyrimidinyl group, a pyridazinyl group, a triadinyl group, an indolyl group, a quinolinyl group, an acridinyl group, a pyrrolidinyl group, a dioxanyl group, a piperidinyl group, a morpholyl group, a piperazinyl group, a triadinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzimidazolyl group, a benzofuranyl group, a dibenzofuranyl group, and the like. The heterocyclic group is preferably a heterocyclic group having 5 to 20 ring atoms, and more preferably a heterocyclic group having 5 to 12 ring atoms.

Examples of the arylene group having 6 to 30 ring carbon atoms represented by L₁ include a divalent residue of the aromatic hydrocarbon ring group having 6 to 30 ring carbon atoms represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, R₈₁ to R₈₇, Ar₁₁, and Ar₁₂. The arylene group is preferably an arylene group having 6 to 20 ring carbon atoms, and more preferably an arylene group having 6 to 12 ring carbon atoms.

Examples of the divalent heterocyclic group having 5 to 30 ring atoms represented by L₁ include a divalent residue of the heterocyclic group having 5 to 30 ring carbon atoms represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, R₈₁ to R₈₇, Ar₁₁, and Ar₁₂.

The 5-membered ring represented by A and B is a cyclic structure that includes a nitrogen atom. Examples of the 5-membered ring represented by A and B include pyrrole, imidazole, oxazole, thiazole, pyrazole, isoxazole, isothiazole, and the like.
The 6-membered ring represented by A and B is a cyclic structure that includes a nitrogen atom. Examples of the 6-membered ring represented by A and B include pyridine, pyridazine, pyrimidine, pyrazine, piperidine, piperazine, and the like.

A substituent that may substitute the substituents represented by R₁₁ to R₁₅, R₁₇, R₁₈, R₂₁ to R₂₆, R₃₁ to R₃₆, R₄₁ to R₄₅, R₅₁ to R₅₅, R₆₂ to R₆₆, R₇₁ to R₇₇, R₈₁ to R₈₇, Ar₁₁, Ar₁₂, L₁, and HAr is not particularly limited as long as the effects of the invention can be achieved. Examples of such a substituent include a halogen atom, an amino group, a cyano group, an alkyl group, an alkenyl group, a cycloalkyl group, an alkoxy group, an aromatic hydrocarbon ring group, a heterocyclic group, an aralkyl group, an aryloxy group, and the like. Specific examples of each substituent include those mentioned above in connection with R₁₁ to R₁₅, R₁₇, R₁₈, R₂₁ to R₂₆, R₃₁ to R₃₈, Ar₁₁, Ar₁₂, L₁, and HAr. The substituent is preferably an alkyl group, an aromatic hydrocarbon ring group, a heterocyclic group, or the like.
When each group is substituted with a plurality of substituents, the plurality of substituents may form a ring. It is preferable that the plurality of substituents form a 6-membered aromatic ring.
When fabricating the organic EL device according to the invention by deposition, it is preferable that the substituent be other than a substituent that includes a double bond and does not include only a cyclic structure (e.g., styryl group) from the viewpoint of improving the lifetime.

Note that the term "unsubstituted" used in connection with each substituent of the nitrogen-containing heterocyclic derivative according to the invention refers to substitution with a hydrogen atom. The term "hydrogen atom" used herein includes light hydrogen and deuterium.

Specific examples of the heterocyclic derivative shown by the formula (3) are shown below.

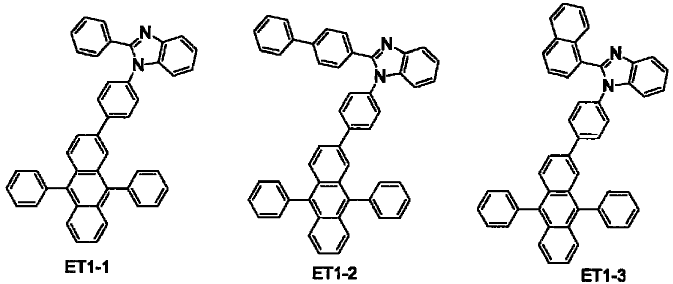

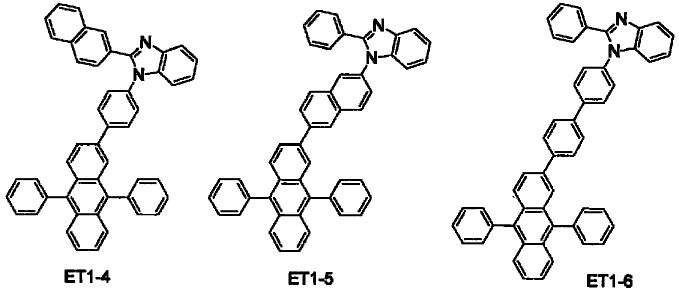

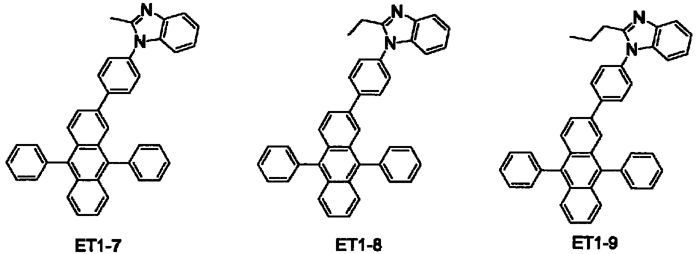

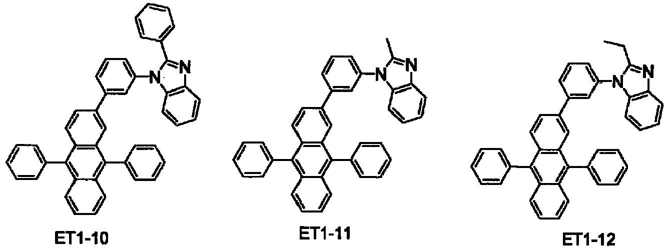

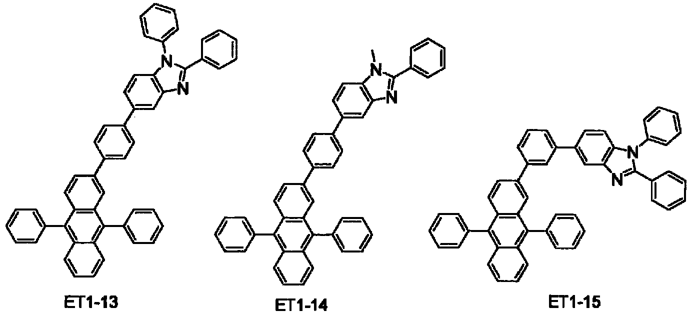

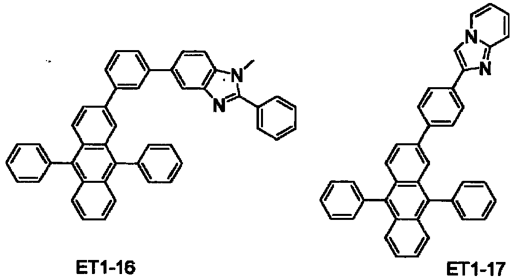

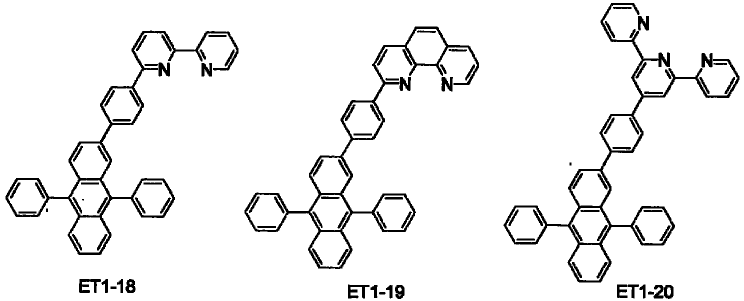

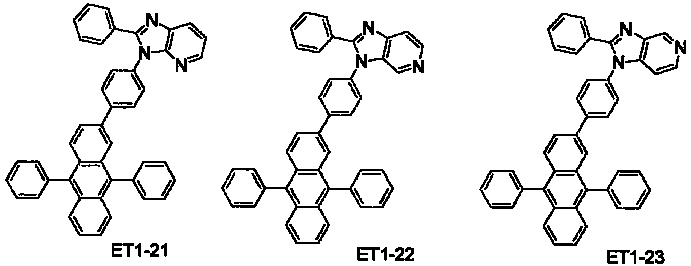

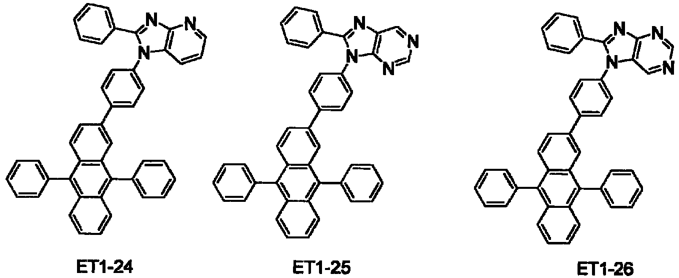

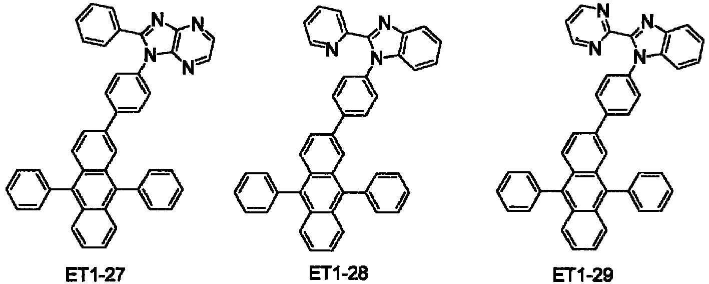

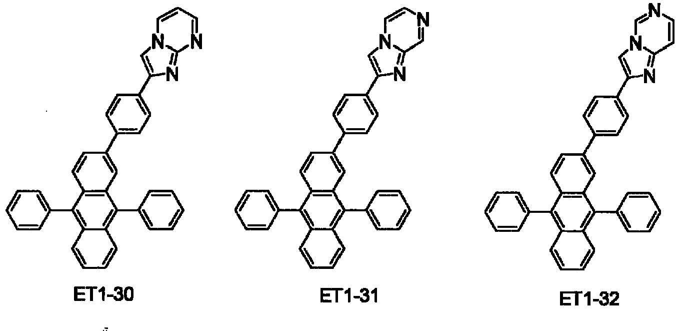

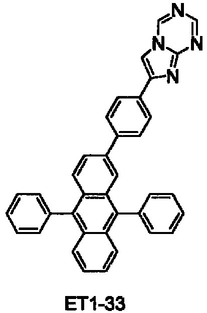

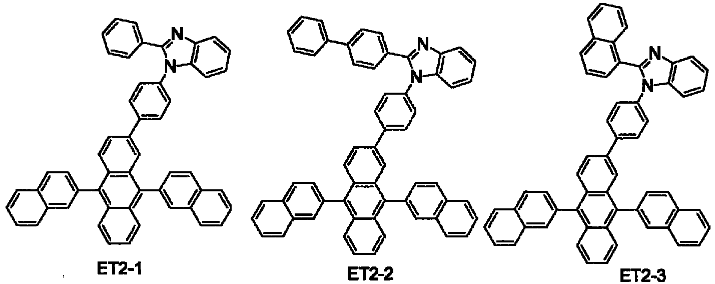

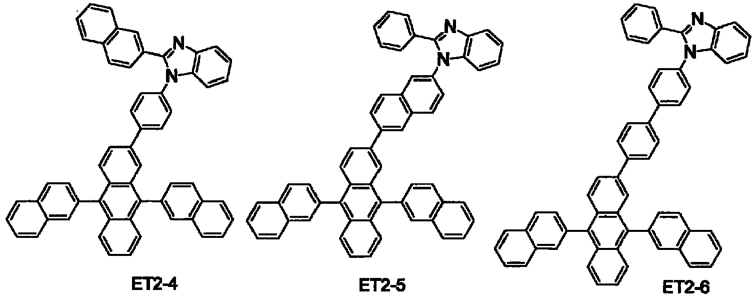

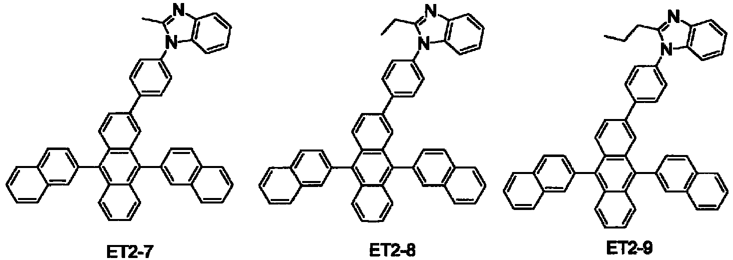

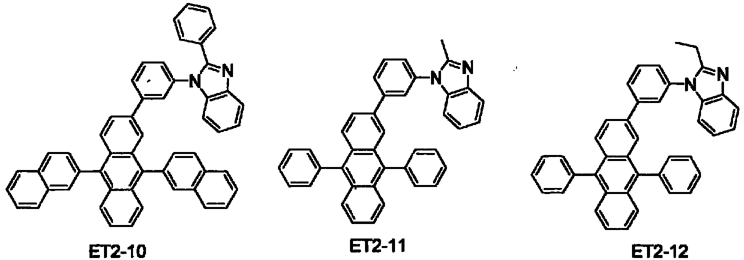

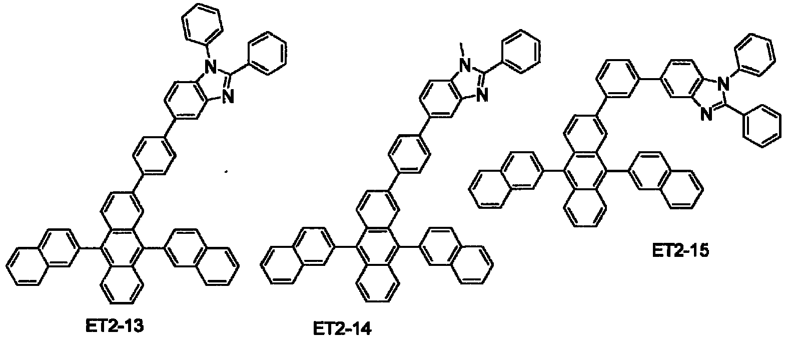

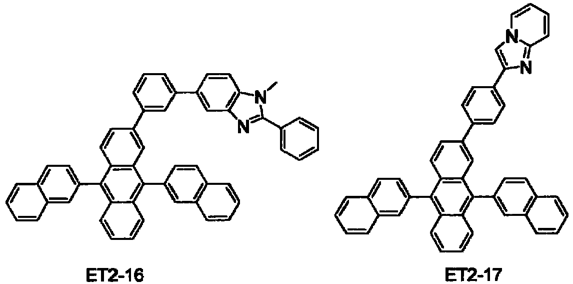

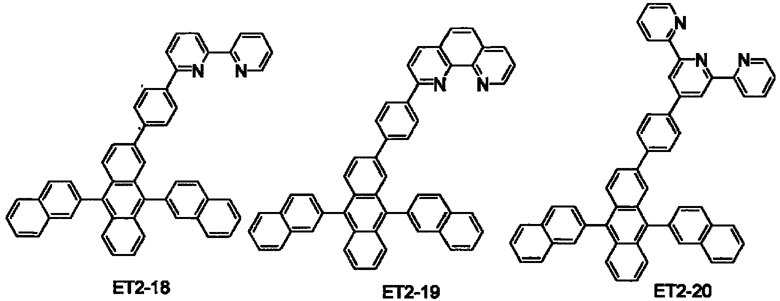

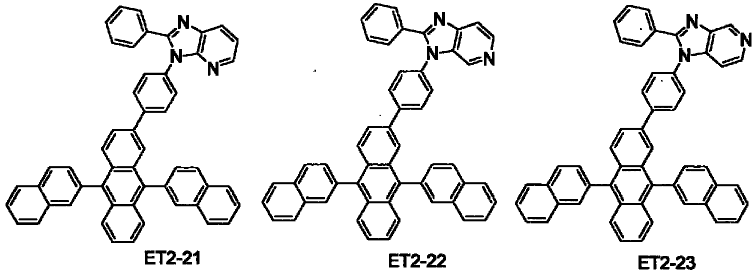

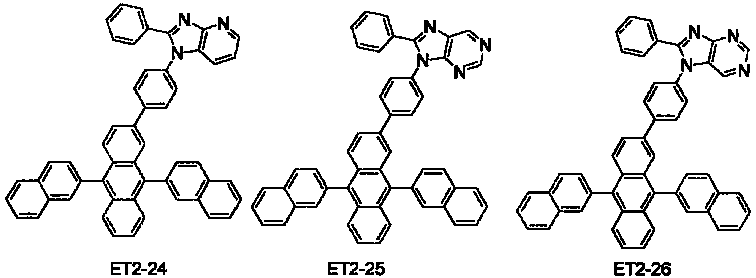

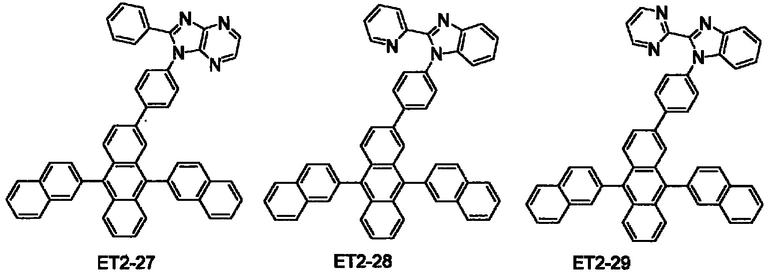

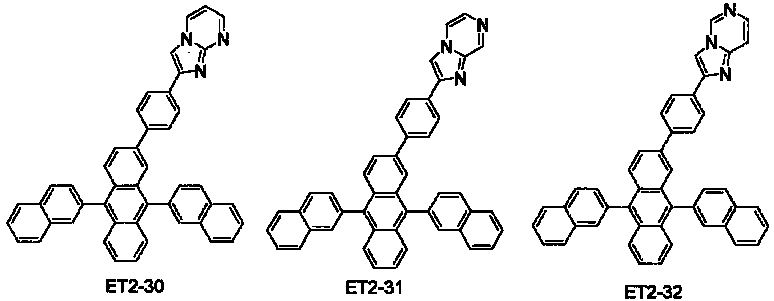

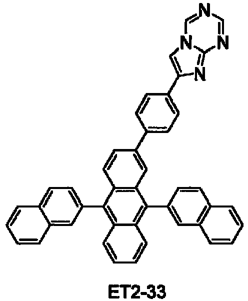

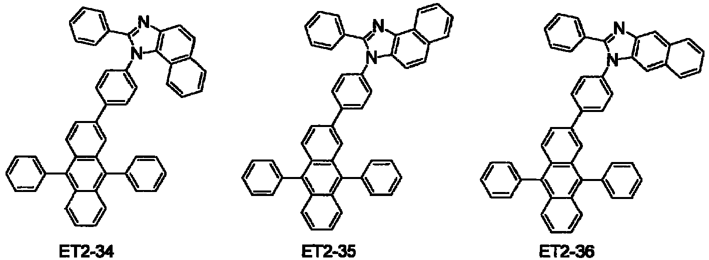

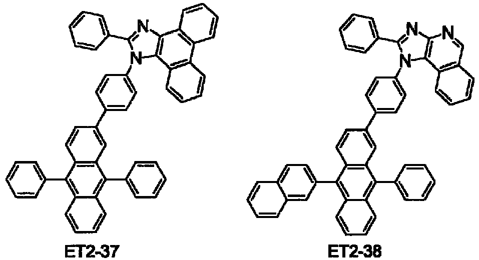

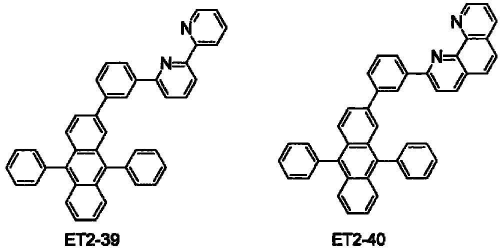

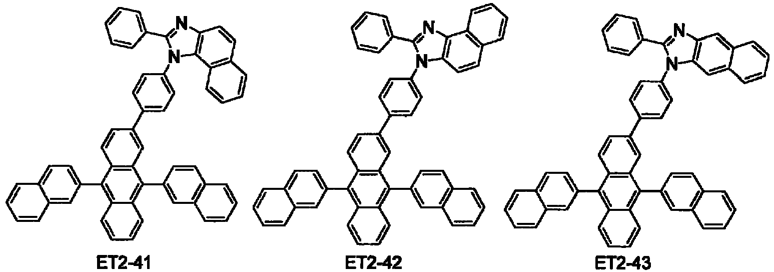

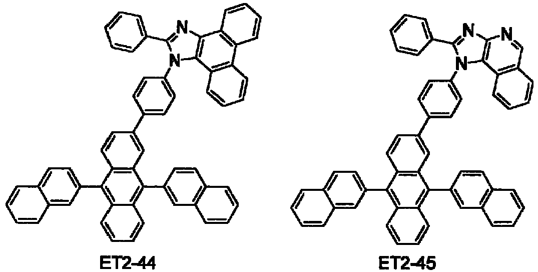

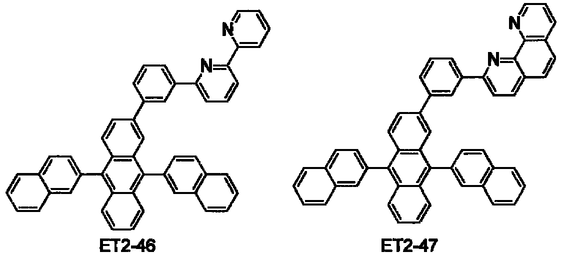

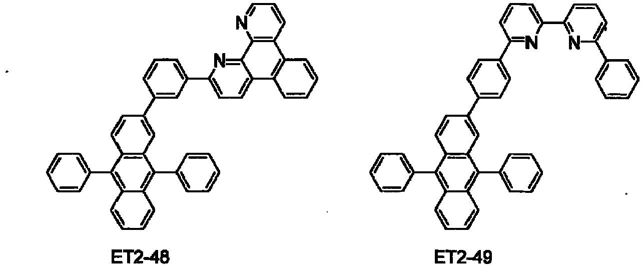

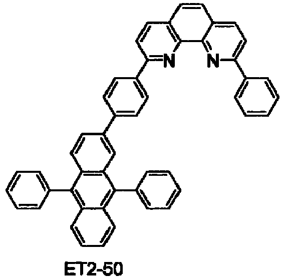

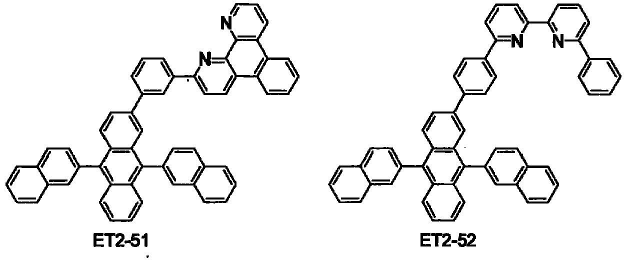

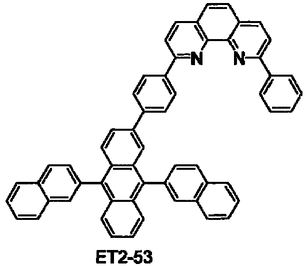

It is preferable that the second organic layer further include a reducing dopant. It is more preferable that the second organic layer be doped with a reducing dopant such as an alkali metal near the cathode-side interface of the second organic layer in order to facilitate acceptance of electrons from the cathode.
Examples of the reducing dopant include donor metals, donor metal compounds, and donor metal complexes. These reducing dopants may be used either individually or in combination.

The term "donor metal" used herein refers to a metal having a work function of 3.8 eV or less. The donor metal is preferably an alkali metal, an alkaline-earth metal, or a rare earth metal, and more preferably Cs, Li, Na, Sr, K, Mg, Ca, Ba, Yb, Eu, or Ce.
The term "donor metal compound" used herein refers to a compound that includes a donor metal. The donor metal compound is preferably a compound that includes an alkali metal, an alkaline-earth metal, or a rare earth metal, and more preferably a halide, an oxide, a carbonate, or a borate of these metals. For example, the donor metal compound is a compound shown by MOₓ (wherein M is a donor metal, and x is a number from 0.5 to 1.5), MFₓ (x is a number from 1 to 3), or M(CO₃)ₓ (x is a number from 0.5 to 1.5).

The term "donor metal complex" refers to a complex of a donor metal. The donor metal complex is preferably an organic complex of an alkali metal, an alkaline-earth metal, or a rare earth metal. The donor metal complex is preferably an organic metal complex shown by the following formula (I).

M(̵Q)ₙ (I)

wherein M is a donor metal, Q is a ligand (preferably a carboxylic acid derivative, a diketone derivative, or a quinolinic derivative), and n is an integer from 1 to 4.

Specific examples of the donor metal complex include the tungsten paddlewheel disclosed in JP-A-2005-72012, and the like. The phthalocyanine compound disclosed in JP-A-H11-345687 in which the central metal is an alkali metal or an alkaline-earth metal, may also be used as the donor metal complex.

The organic electroluminescence device according to the invention preferably includes a layer between the second organic layer and the cathode, the layer being formed of one or more substances selected from the group consisting of alkali metals, alkaline-earth metals, rare earth metals, alkali metal oxides, alkali metal halides, alkaline-earth metal oxides, alkaline-earth metal halides, rare earth metal oxides, rare earth metal halides, organic complexes of alkali metals, organic complexes of alkaline-earth metals, and organic complexes of rare earth metals.

Examples of a preferable substance include those mentioned above in connection with the reducing dopant. The same effect as that obtained by adding a reducing dopant to the second organic layer can be obtained by providing a layer that is formed of a reducing dopant material.

### First organic layer

In the organic EL device according to the invention, the first organic layer is provided between the anode and the second organic layer, and may function as an emitting layer, for example.
When the first organic layer functions as an emitting layer, the monoanthracene derivative shown by the formula (1) is a host material, and the fused aromatic amine derivative shown by the formula (2) is a dopant material.
The content of the monoanthracene derivative shown by the formula (1) in the first organic layer is 50 to 99.9%, and preferably 90 to 99%, for example. The content of the fused aromatic amine derivative shown by the formula (2) in the first organic layer is 0.1 to 50%, and preferably 1 to 10%, for example.

The monoanthracene derivative shown by the formula (1) is a compound that includes one - anthracene ring in the molecule.
Ar₁ and Ar₂ in the monoanthracene derivative shown by the formula (1) are independently a substituted or unsubstituted aromatic hydrocarbon ring group or a substituted or unsubstituted heterocyclic group. Specific examples of the aromatic hydrocarbon ring group and the heterocyclic group include those mentioned above. It is preferable that Ar₁ and Ar₂ be independently a substituted or unsubstituted group formed of 1 to 4 aromatic hydrocarbon rings or heterocyclic rings.

Examples of the group formed of 1 to 4 aromatic hydrocarbon rings include a monovalent substituent selected from the group consisting of a benzene ring, a naphthalene ring, a phenanthrene ring, a fluorene ring, a benzanthracene ring, and a benzophenanthrene ring, and a monovalent substituent in which 2 to 4 aromatic hydrocarbon rings selected from the group consisting of these aromatic hydrocarbon rings are bonded via a single bond. A benzene ring is preferable as the 1 to 4 aromatic hydrocarbon rings.
The group formed of 1 to 4 aromatic hydrocarbon rings is formed of a benzene ring, a naphthalene ring, or a combination of a benzene ring and a naphthalene ring, for example. Specific examples of the group formed of 1 to 4 aromatic hydrocarbon rings include a phenyl group, a 1-naphthyl group, a 2-naphthyl group, a 1-naphthylphenyl group, a 2-naphthylphenyl group, a 9-phenanthryl group, a 9-phenanthrylphenyl group, and the like.
Examples of the group formed of 1 to 4 heterocyclic rings include a benzothiophenyl group, a dibenzothiophenyl group, a benzofuranyl group, a dibenzofuranyl group, a carbazolyl group, and the like.
Examples of a substituent that may substitute the group formed of 1 to 4 aromatic hydrocarbon rings or heterocyclic rings include an alkyl group having 1 to 4 carbon atoms, a silyl group, a silyl group substituted with an alkyl group having 1 to 4 carbon atoms, a cyano group, a halogen atom, an alkoxy group, and the like.

It is preferable that Ar₁ and Ar₂ be independently (preferably both of Ar₁ and Ar₂) a substituted or unsubstituted fused aromatic hydrocarbon ring group having 10 to 30 ring carbon atoms.
The term "fused aromatic hydrocarbon ring group" used herein refers to a substituent that includes only a plurality of aromatic rings (fused ring). The fused ring is preferably naphthalene, phenanthrene, fluorene, benzanthracene, or benzophenanthrene.

It is also preferable that Ar₁ in the formula (1) be a substituted or unsubstituted phenyl group, and Ar₂ be a substituted or unsubstituted fused aromatic hydrocarbon ring group having 10 to 30 ring carbon atoms, or Ar₁ and Ar₂ be independently a substituted or unsubstituted phenyl group. It is preferable that Ar₁ be a substituted phenyl group. It is preferable that Ar₂ be a substituted or unsubstituted fused aromatic hydrocarbon ring group having 10 to 20 ring carbon atoms, and more preferably substituted or unsubstituted naphthalene, phenanthrene, fluorene, benzanthracene, or benzophenanthrene.

A substituent that may substitute Ar₁ and Ar₂ is preferably an aromatic hydrocarbon ring group having 6 to 30 ring carbon atoms or a heterocyclic group having 5 to 30 ring atoms, and more preferably a phenyl group, a 1-naphthyl group, a 2-naphthyl group, a 1-naphthylphenyl group, a 2-naphthylphenyl group, a 9-phenanthryl group, a 9-phenanthrylphenyl group, a benzothiophenyl group, a dibenzothiophenyl group, a benzofuranyl group, a dibenzofuranyl group, or a carbazolyl group.

It is also preferable that Ar₁ and Ar₂ in the anthracene derivative be independently a substituted or unsubstituted phenyl group. A substituent that may substitute the phenyl group is preferably an aromatic hydrocarbon ring group having 6 to 30 ring carbon atoms, or a heterocyclic group having 5 to 30 ring atoms, an alkyl group having 1 to 4 carbon atoms, a silyl group, a silyl group substituted with an alkyl group having 1 to 4 carbon atoms, a cyano group, a halogen atom, an alkoxy group, or the like, among them, preferably an aromatic hydrocarbon ring group having 6 to 30 ring carbon atoms or a heterocyclic group having 5 to 30 ring atoms, and more preferably a phenyl group, a 1-naphthyl group, a 2-naphthyl group, a 1-naphthylphenyl group, a 2-naphthylphenyl group, a 9-phenanthryl group, a 9-phenanthrylphenyl group, a benzothiophenyl group, a dibenzothiophenyl group, a benzofuranyl group, a dibenzofuranyl group, or a carbazolyl group.

It is preferable that R₁ to R₈ be independently a hydrogen atom, a fluorine atom, an alkyl group having 1 to 10 carbon atoms, a substituted or unsubstituted cycloalkyl group having 3 to 10 carbon atoms, a substituted or unsubstituted alkylsilyl group having 3 to 20 carbon atoms, or a substituted or unsubstituted arylsilyl group having 6 to 30 ring carbon atoms, more preferably a hydrogen atom, an alkyl group having 1 to 10 carbon atoms, or a substituted or unsubstituted cycloalkyl group having 3 to 10 carbon atoms, and most preferably a hydrogen atom.

The fused aromatic amine derivative shown by the formula (2) is preferably a compound shown by the following formula (12) or a compound shown by the following formula (13). 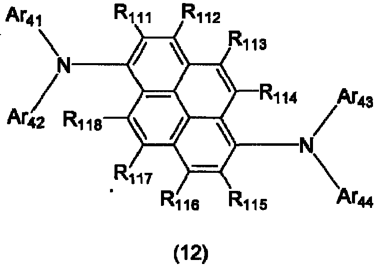 wherein R₁₁₁ to R₁₁₈ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₄₁ to Ar₄₄ are independently a substituted or unsubstituted aromatic hydrocarbon ring group having 6 to 30 ring carbon atoms or a substituted or unsubstituted heterocyclic group having 5 to 30 ring atoms. 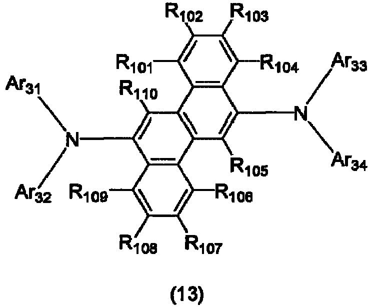 wherein R₁₀₁, to R₁₁₀ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₃₁ to Ar₃₄ are independently a substituted or unsubstituted aromatic hydrocarbon ring group having 6 to 30 ring carbon atoms or a substituted or unsubstituted heterocyclic group having 5 to 30 ring atoms.

The aromatic hydrocarbon ring group having 6 to 30 ring carbon atoms in the formulas (12) and (13) is preferably a phenyl group, a naphthyl group, a phenanthryl group, a fluorenyl group, or the like, and more preferably a phenyl group or a naphthyl group.

The heterocyclic group having 5 to 30 ring atoms in the formulas (12) and (13) is preferably a pyridyl group, a pyrimidyl group, a triadinyl group, a benzofuranyl group, a benzothiophenyl group, a benzofuranyl group, a dibenzofuranyl group, a carbazolyl group, or the like, and more preferably a pyridyl group, a pyrimidyl group, a carbazolyl group, a dibenzofuranyl group, or a dibenzothiophenyl group. The aromatic hydrocarbon ring group and the heterocyclic group may be substituted with a substituent. Examples of a preferable substituent include an alkyl group having 1 to 10 carbon atoms, an alkoxy group having 1 to 10 carbon atoms, an alkylsilyl group having 3 to 10 carbon atoms, a cyano group, a halogen atom, and an aromatic hydrocarbon ring group having 6 to 30 carbon atoms. Specific examples of these groups or atoms include those mentioned above.

It is preferable that Ar₄₁ and Ar₄₄ in the formula (12) be independently a substituted phenyl group. Examples of a preferable substituent include an alkyl group, a halogen atom, an aromatic hydrocarbon ring group, an alkylsilyl group, and a cyano group.

When the fused aromatic amine derivative shown by the formula (2) is the compound shown by the formula (12), it is preferable that at least one of R₁₁₂ and R₁₁₆ be a substituted or unsubstituted alkyl group having 1 to 10 carbon atoms or a substituted or unsubstituted cycloalkyl group having 3 to 10 carbon atoms. It is more preferable that at least one of R₁₁₂ and R₁₁₆ be a substituted or unsubstituted alkyl group having 1 to 10 carbon atoms.

The compound shown by the formula (12) is preferably a compound shown by the following formula (12-1). 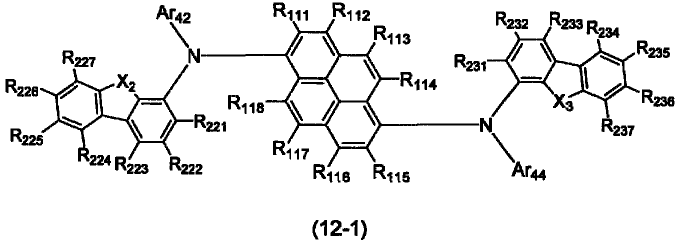 wherein R₁₁₁ to R₁₁₈, Ar₄₂, and Ar₄₄ are the same as defined for the formula (12), R₂₂₁ to R₂₂₇ and R₂₃₁ to R₂₃₇ are independently a hydrogen atom, a halogen atom, a substituted or unsubstituted alkyl group having 1 to 10 carbon atoms, a substituted or unsubstituted alkenyl group having 2 to 20 carbon atoms, a substituted or unsubstituted alkynyl group having 2 to 20 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, a cyano group, a substituted or unsubstituted aromatic hydrocarbon ring group having 6 to 30 ring carbon atoms, or a substituted or unsubstituted heterocyclic group having 5 to 30 ring atoms, provided that adjacent substituents among R₂₂₁ to R₂₂₇ and R₂₃₁ to R₂₃₇ may form a saturated or unsaturated ring, and X₂ and X₃ are independently an oxygen atom or a sulfur atom.

R₁₁₁ to R₁₁₈ in the formula (12-1) are preferably hydrogen atoms. It is also preferable that R₁₁₂ be a substituted or unsubstituted alkyl group having 1 to 10 carbon atoms, a substituted or unsubstituted cycloalkyl group having 3 to 10 carbon atoms, or a substituted or unsubstituted alkylsilyl group having 3 to 20 carbon atoms, and R₁₁₁ and R₁₁₃ to R₁₁₈ be hydrogen atoms. It is also preferable that R₁₁₂ and R₁₁₆ be a substituted or unsubstituted alkyl group having 1 to 10 carbon atoms, a substituted or unsubstituted cycloalkyl group having 3 to 10 carbon atoms, or a substituted or unsubstituted alkylsilyl group having 3 to 20 carbon atoms, and R₁₁₁, R₁₁₃, R₁₁₄, R₁₁₅, R₁₁₇, and R₁₁₈ be hydrogen atoms. The substituted or unsubstituted alkyl group having 1 to 10 carbon atoms represented by R₁₁₂ and R₁₁₆ is preferably an alkyl group having 1 to 6 carbon atoms.

X₁, X₂, and X₃ in the formula (12-1) are preferably oxygen atoms.

It is also preferable that the compound shown by the formula (12) be a compound shown by the following formula (12-2). 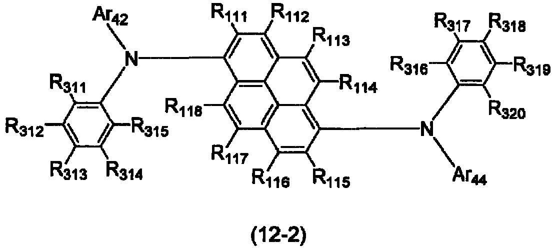 wherein R₁₁₁ to R₁₁₈, Ar₄₂, and Ar₄₄ are the same as defined for the formula (12), R₃₁₁ to R₃₂₀ are independently a hydrogen atom, a halogen atom, a substituted or unsubstituted alkyl group having 1 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aromatic hydrocarbon ring group having 6 to 30 ring carbon atoms, a substituted or unsubstituted heterocyclic group having 5 to 30 ring atoms, or an electron-withdrawing group, provided that at least one of R₃₁₁ to R₃₁₅ is the electron-withdrawing group, and at least one of R₃₁₆ to R₃₂₀ is the electron-withdrawing group.

Examples of the electron-withdrawing group include a cyano group, a fluorine atom, an alkyl halide group, an alkyl halide-substituted alkyl group, a nitro group, a carbonyl group, and the like. Among these, a cyano group, a fluorine atom, an alkyl halide group, and an alkyl halide-substituted alkyl group are preferable, and a cyano group is particularly preferable.

It is preferable that one of R₃₁₁ to R₃₁₅ in the formula (12-1) be a cyano group, the remainder of R₃₁₁ to R₃₁₅ be hydrogen atoms, and one of R₃₁₆ to R₃₂₀ be a cyano group, and the remainder of R₃₁₆ to R₃₂₀ be hydrogen atoms.

It is preferable that R₁₁₁, R₁₁₃, R₁₄₁, R₁₁₅, R₁₁₇, and R₁₁₈ in the formula (12-2) be hydrogen atoms. Since R₁₁₂ and R₁₁₆ are active sites of the pyrene ring, the active sites can be protected by introducing a substituent into the active sites. This improves the stability of the compound.
It is particularly preferable that R₁₁₂ and R₁₁₆ be independently a substituted or unsubstituted alkyl group having 1 to 20 carbon atoms, a substituted or unsubstituted cycloalkyl group having 3 to 10 carbon atoms, a substituted or unsubstituted alkylsilyl group having 3 to 20 carbon atoms, or a substituted or unsubstituted arylsilyl group having 6 to 30 carbon atoms. In this case, the emission lifetime is improved.

R₁₀₁ to R₁₁₀ in the formula (13) are preferably hydrogen atoms. It is preferable that Ar₃₁ to Ar₃₄ be substituted with an alkyl group or a cycloalkyl group.

Examples of the groups represented by Ar₁, Ar₂, and R₁ to R₈ in the monoanthracene derivative shown by the formula (1), Ar₂₁, Ar₂₂, Ar₃₁ to Ar₃₄, Ar₄₁ to Ar₄₄, R₁₀₁ to R₁₁₀, and R₁₁₁ to R₁₁₈ in the fused aromatic amine derivatives shown by the formula (2), and a substituent that may substitute these groups include those mentioned above in connection with the formula (3).

Specific examples of the monoanthracene derivative shown by the formula (1) are shown below.

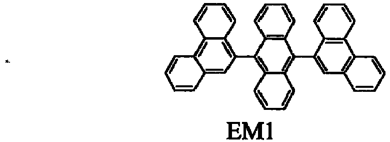

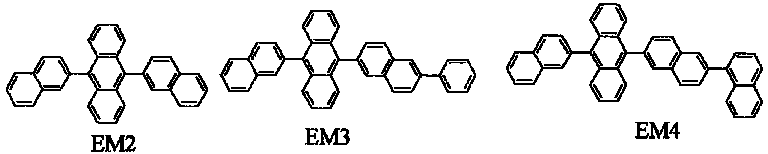

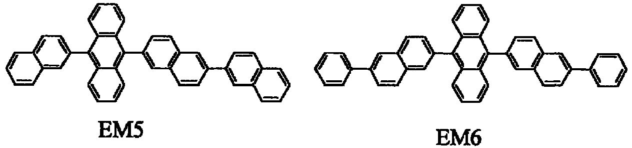

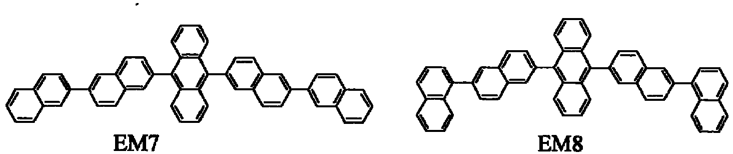

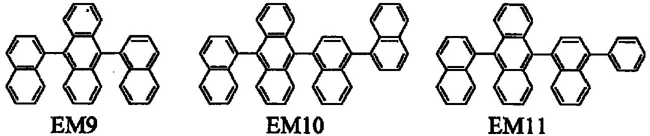

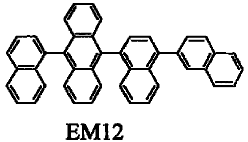

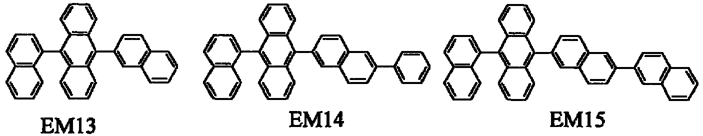

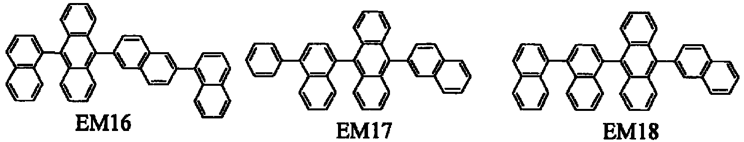

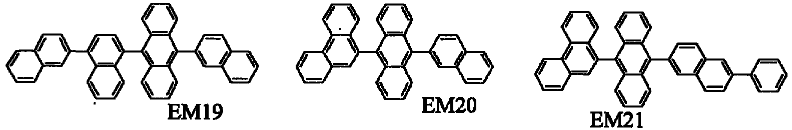

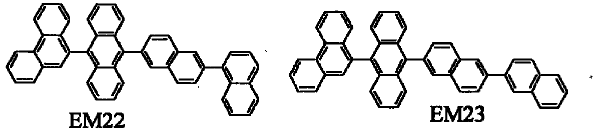

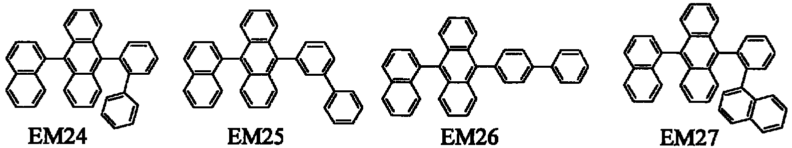

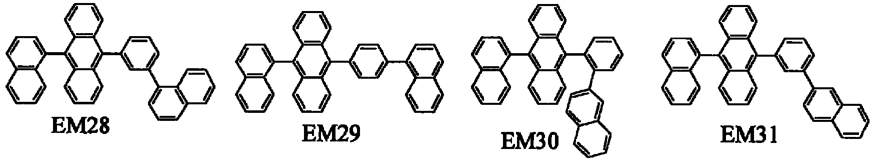

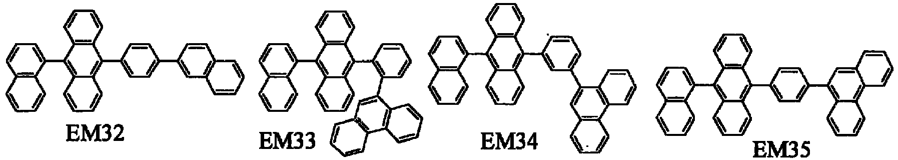

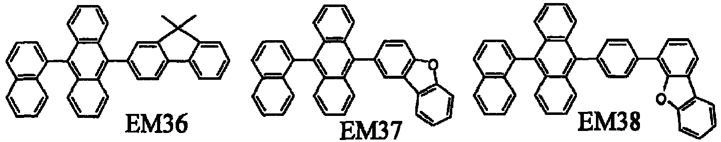

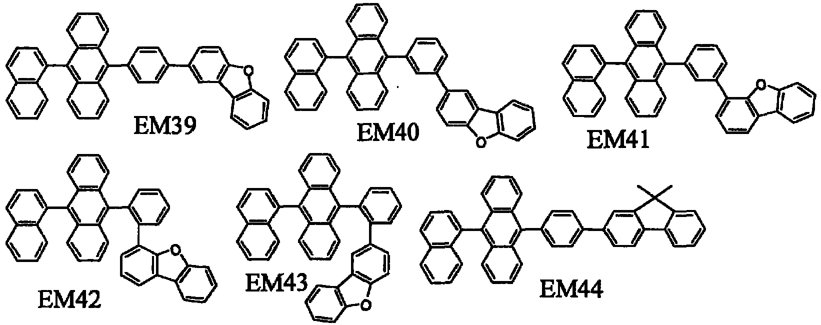

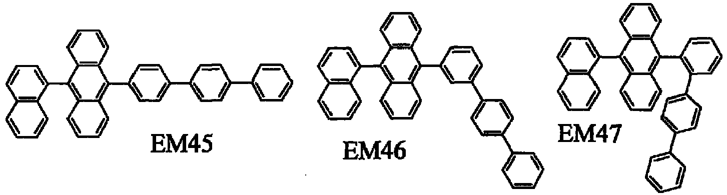

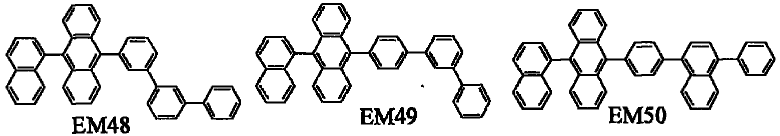

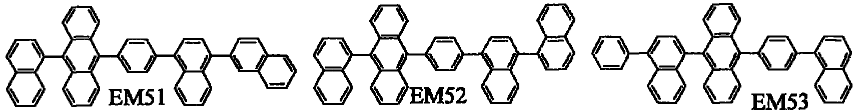

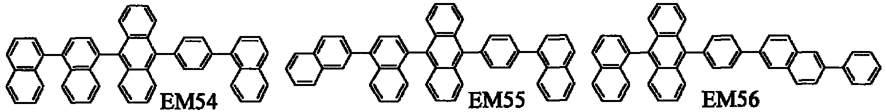

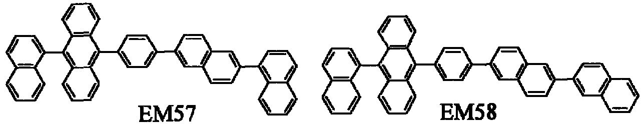

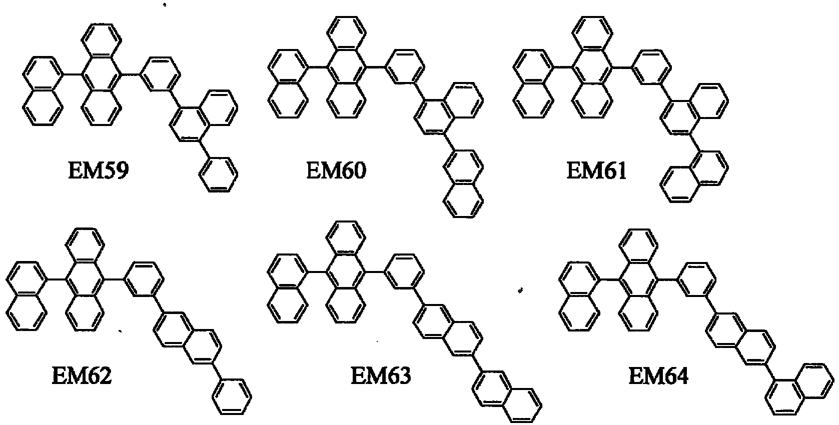

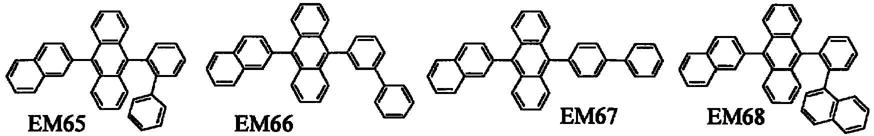

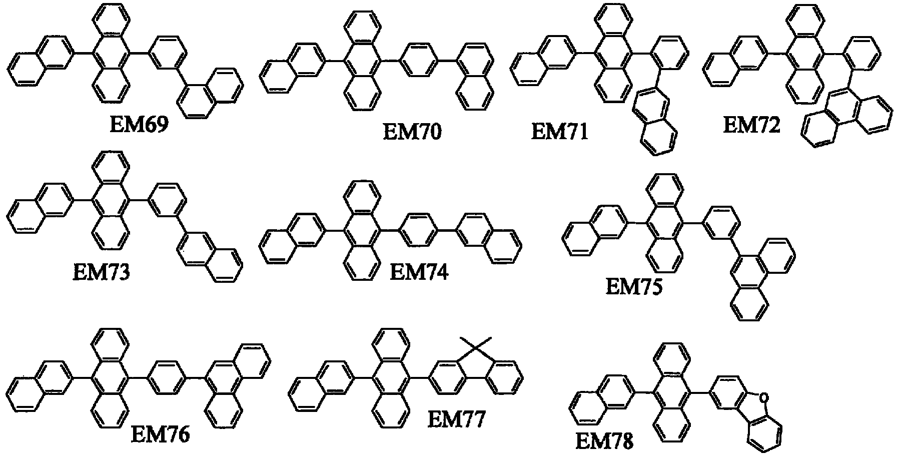

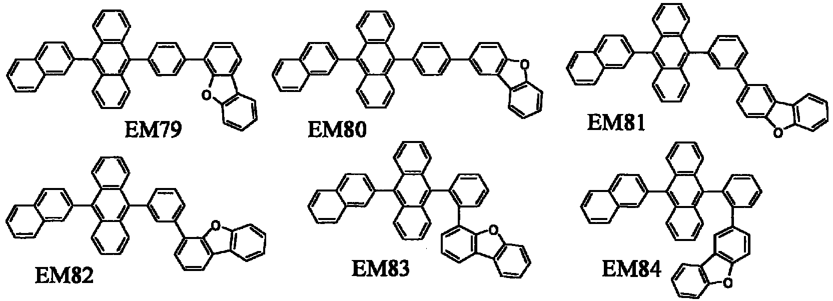

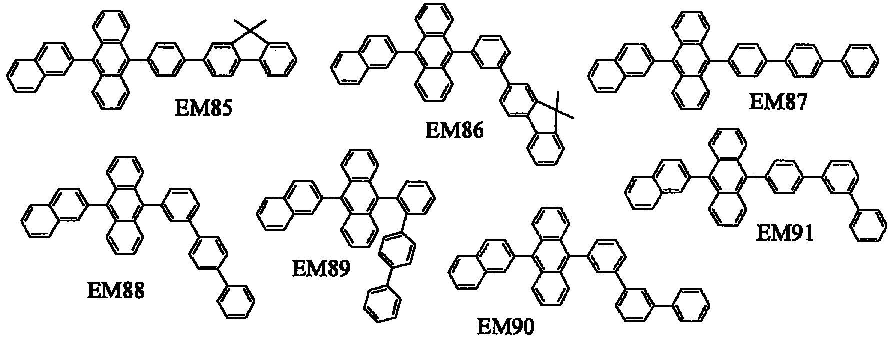

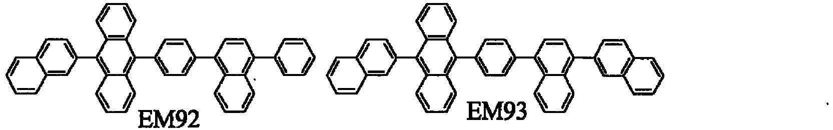

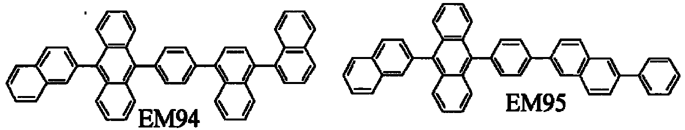

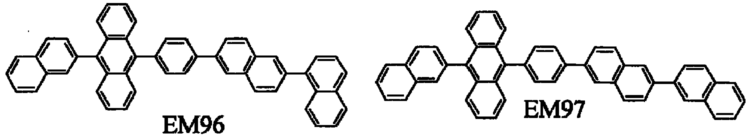

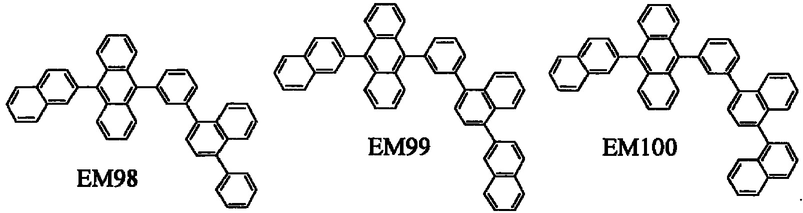

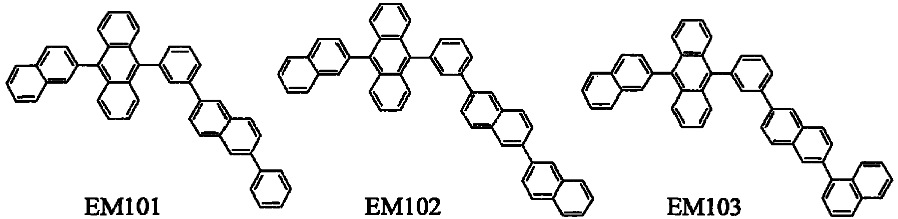

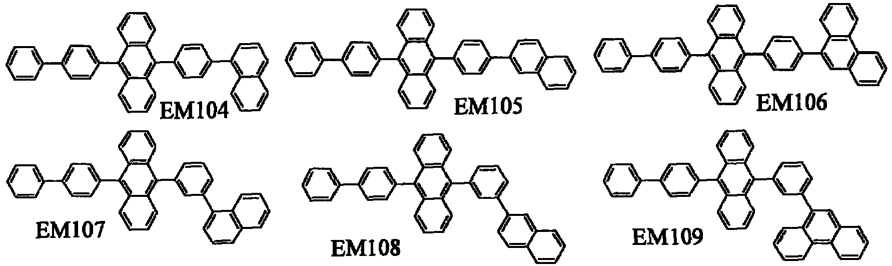

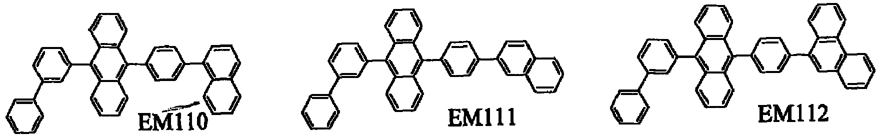

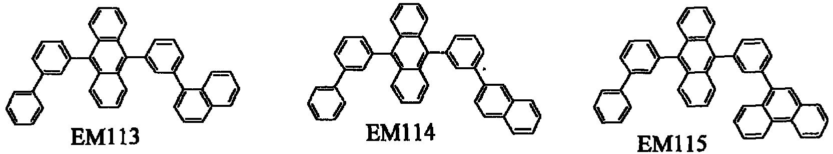

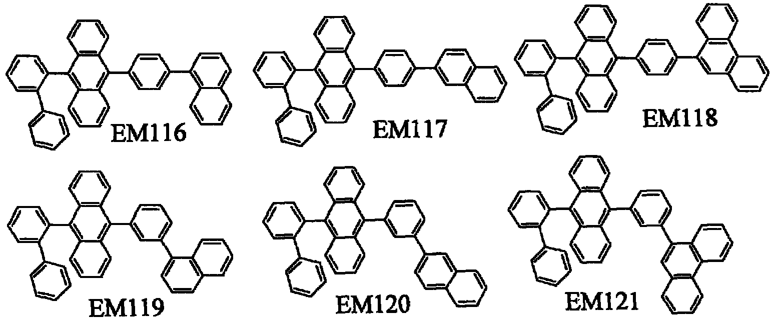

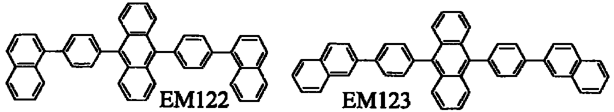

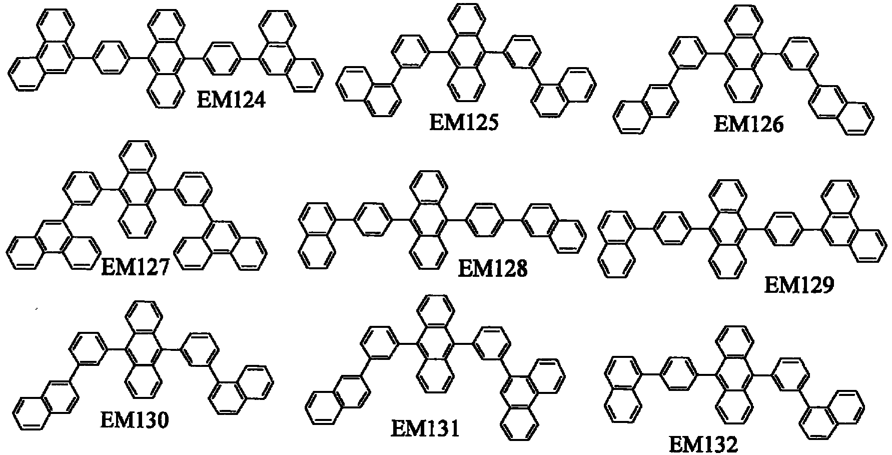

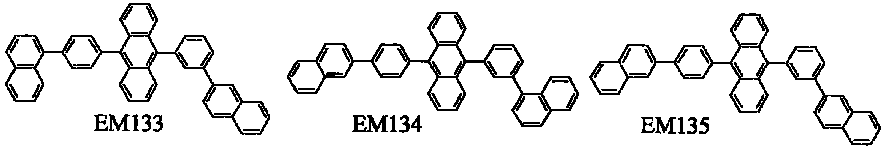

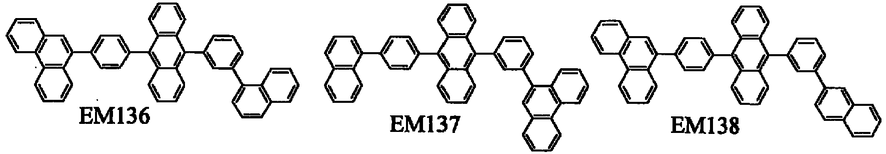

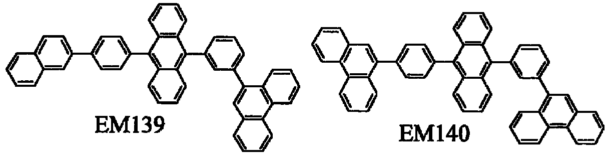

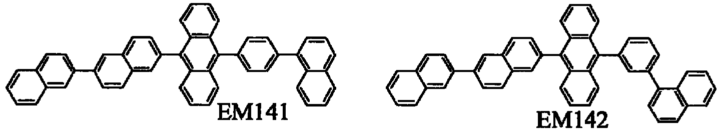

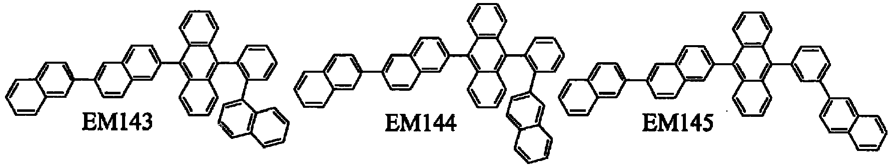

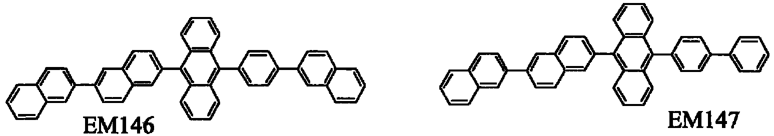

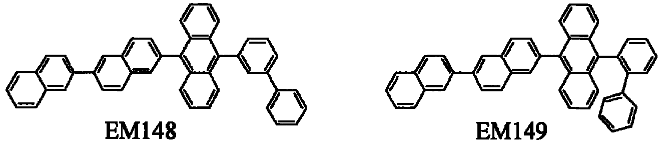

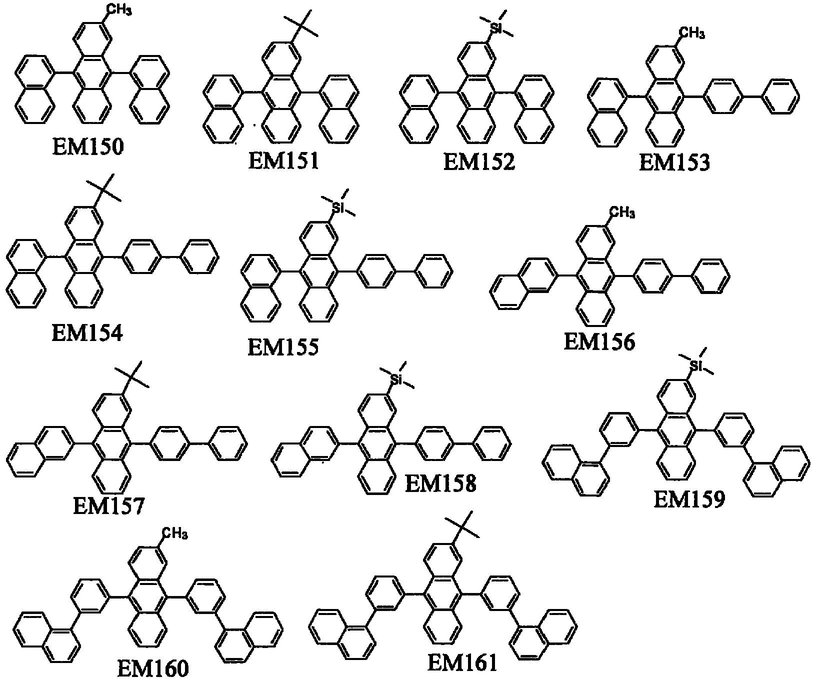

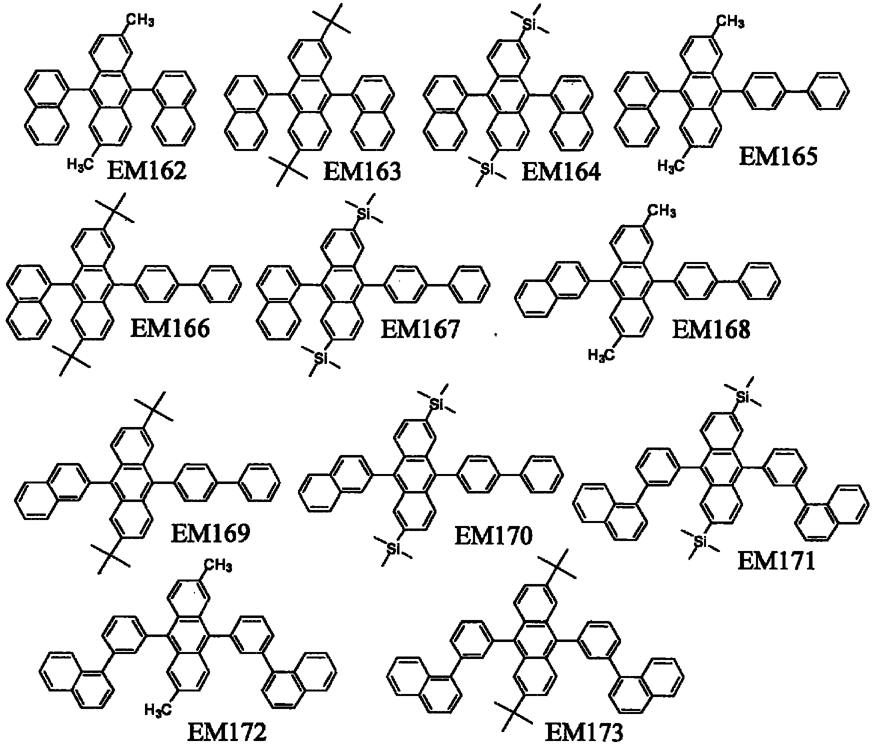

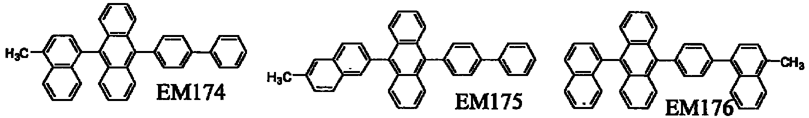

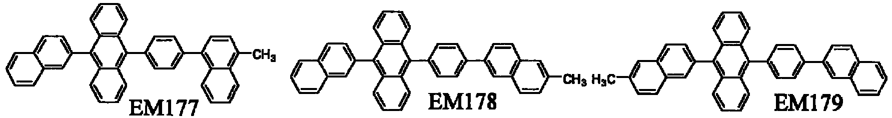

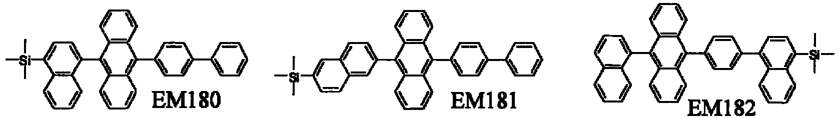

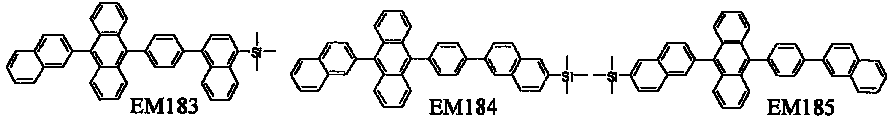

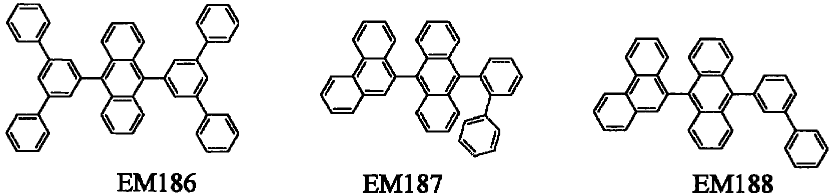

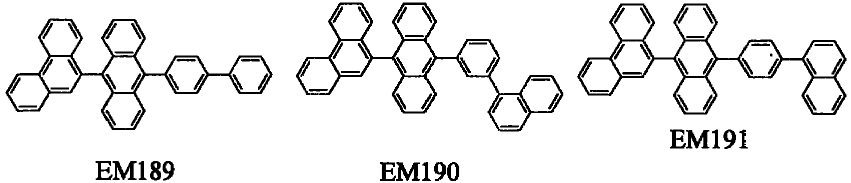

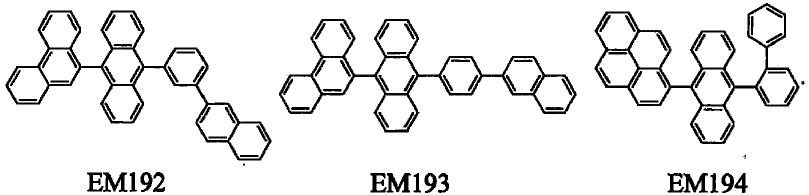

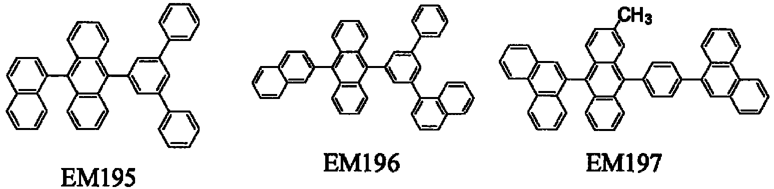

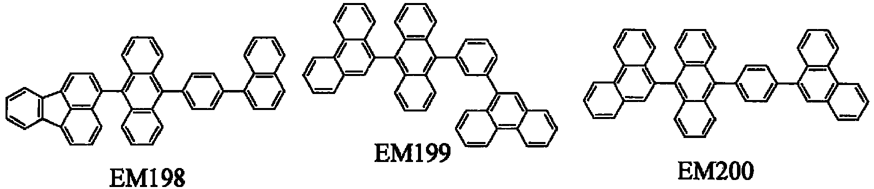

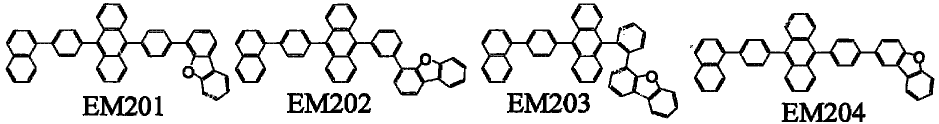

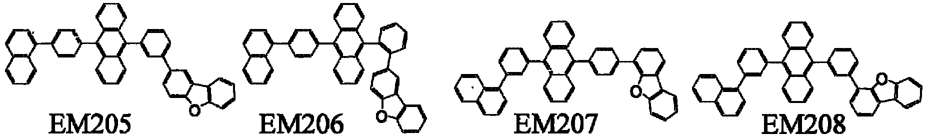

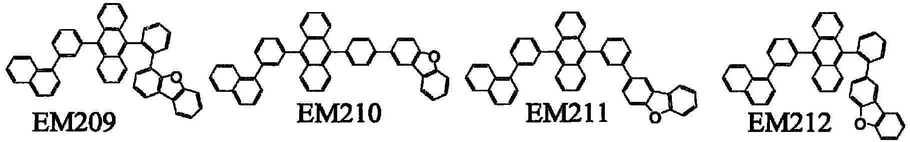

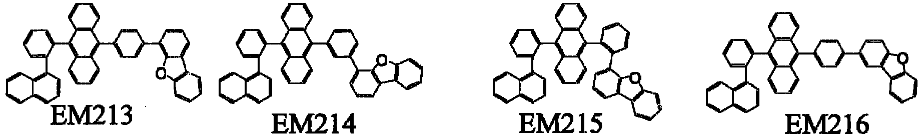

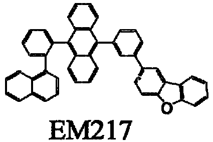

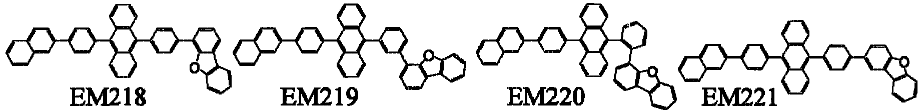

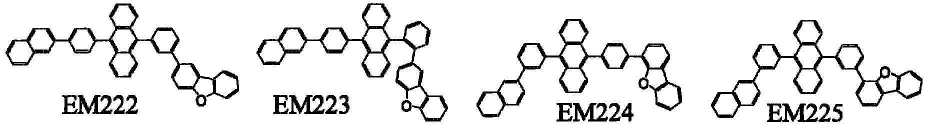

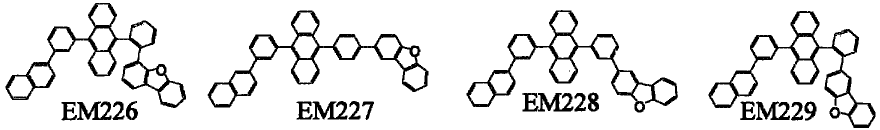

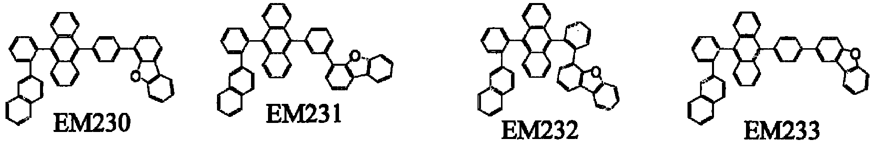

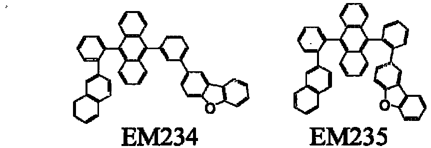

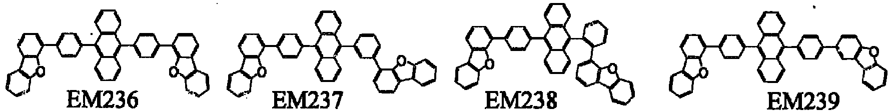

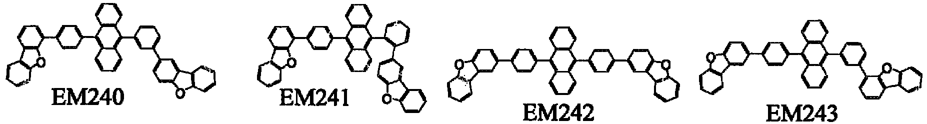

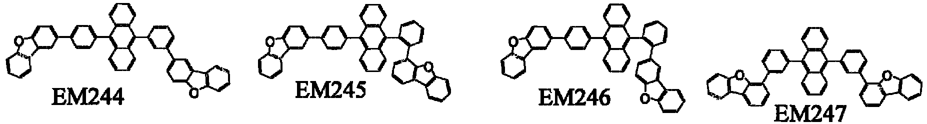

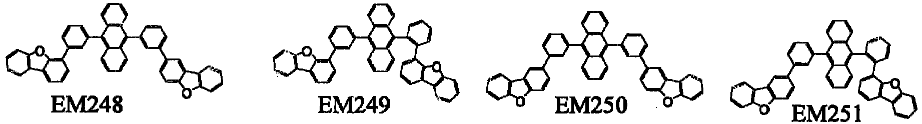

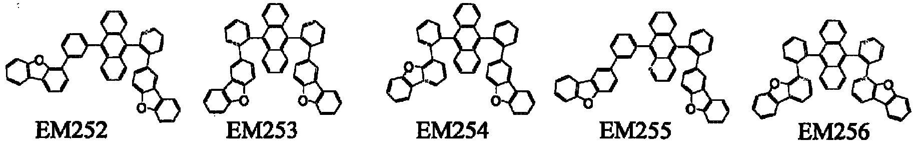

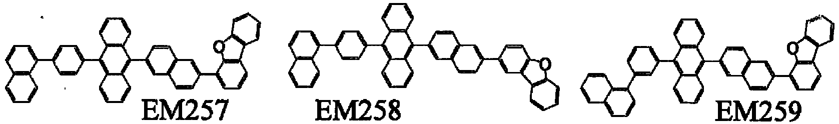

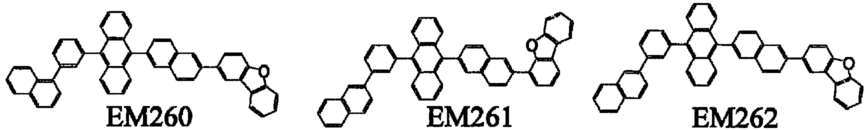

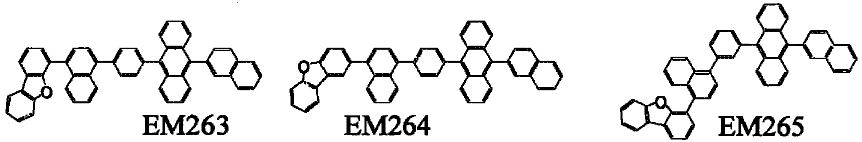

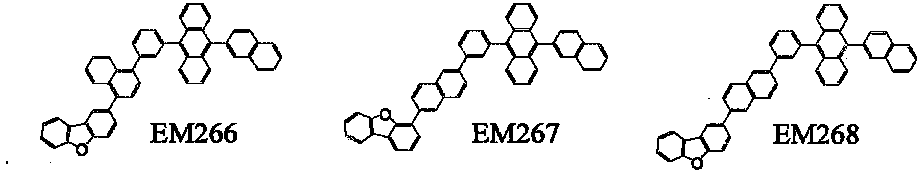

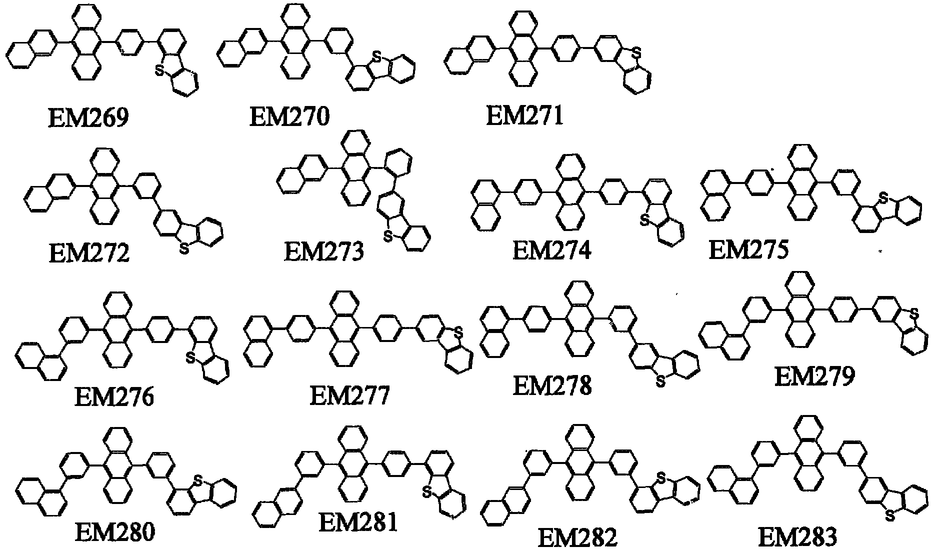

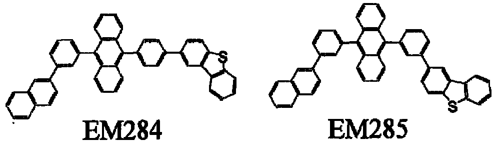

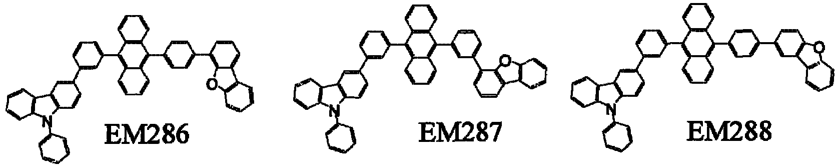

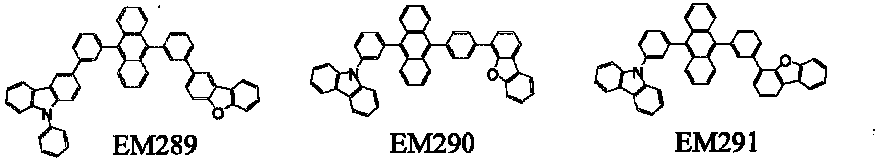

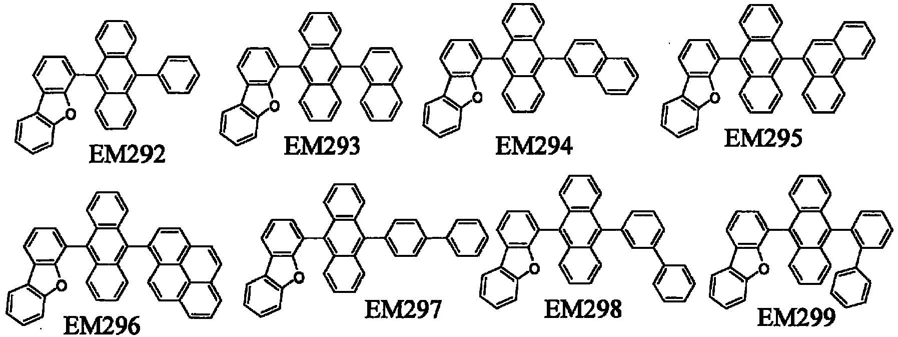

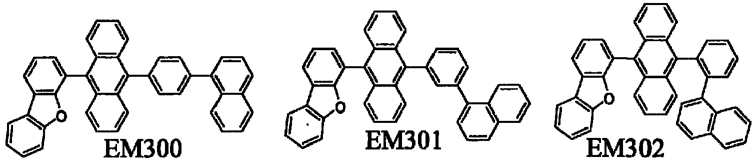

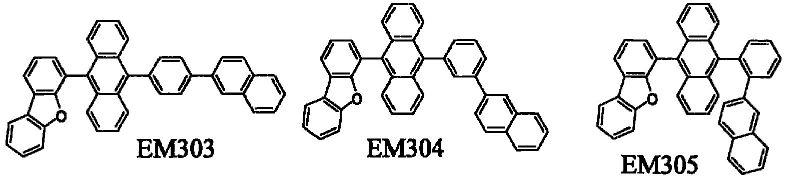

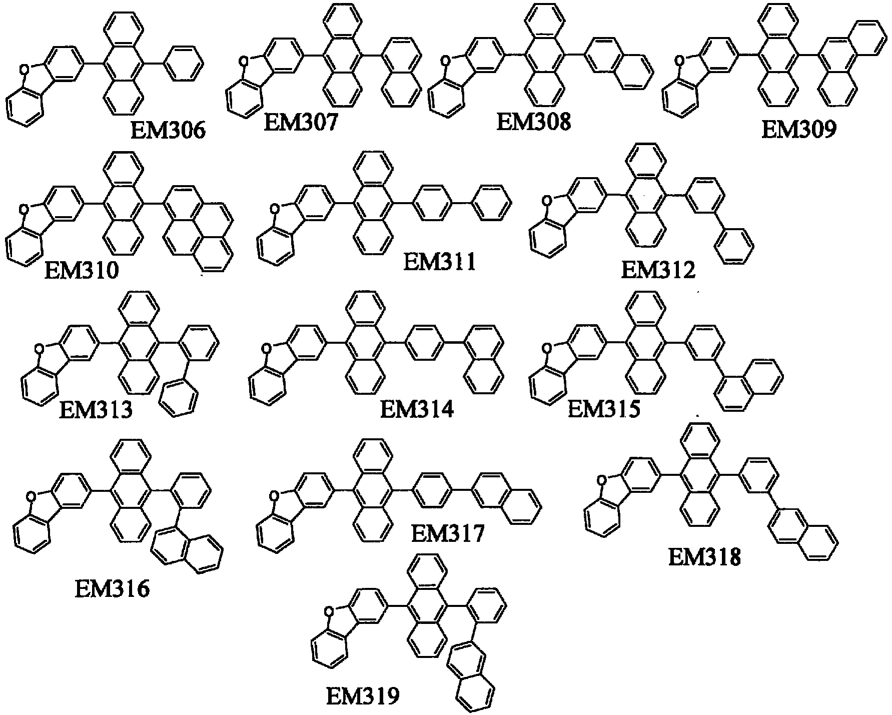

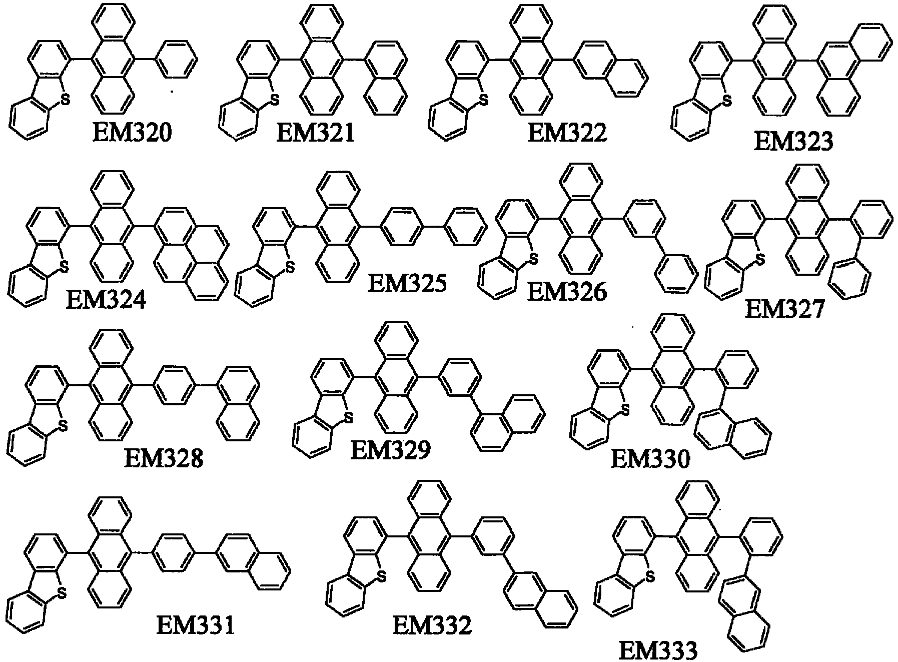

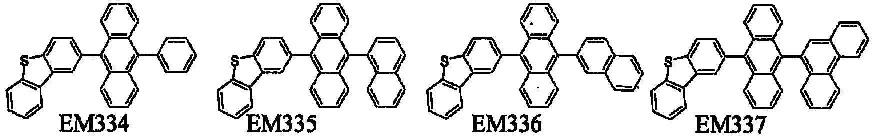

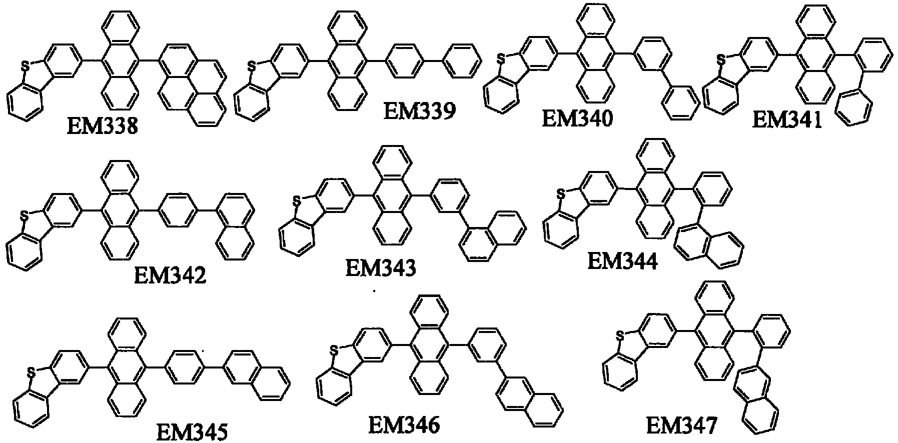

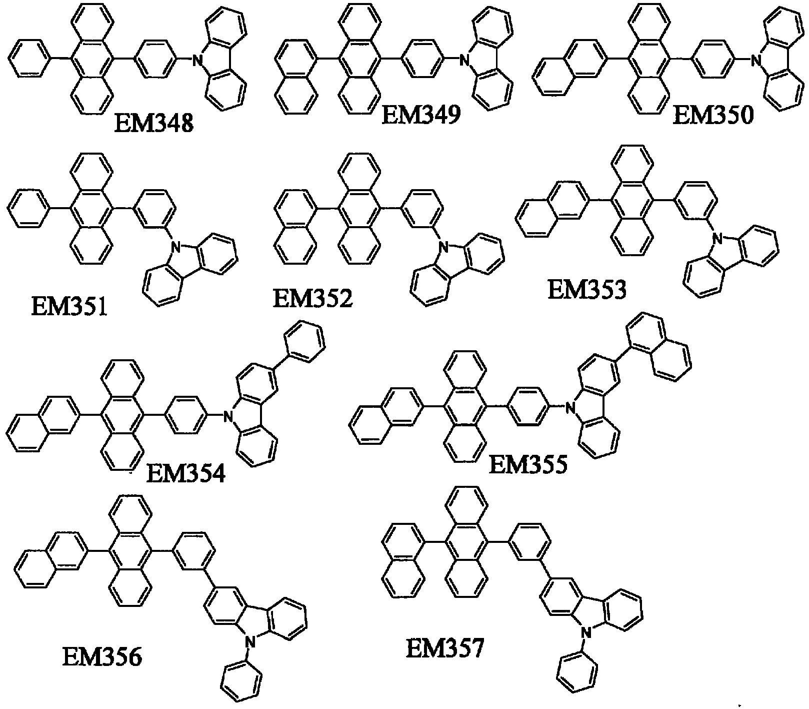

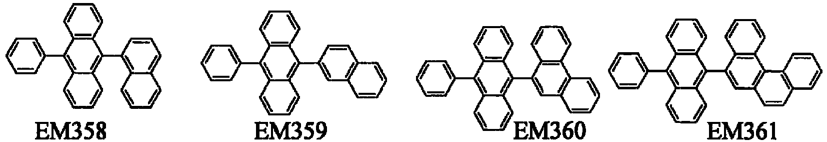

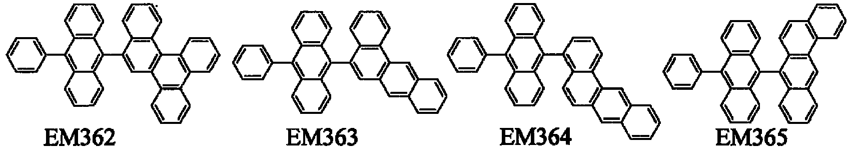

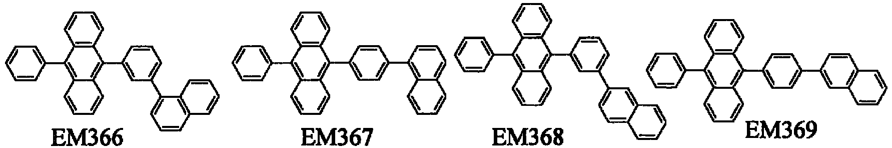

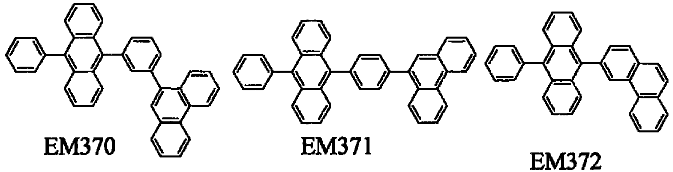

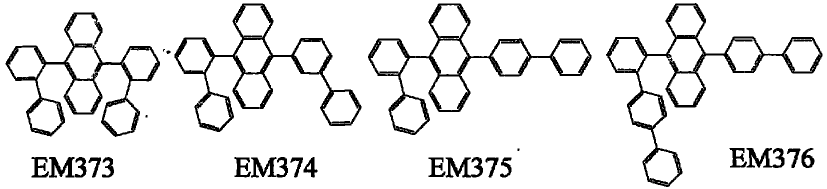

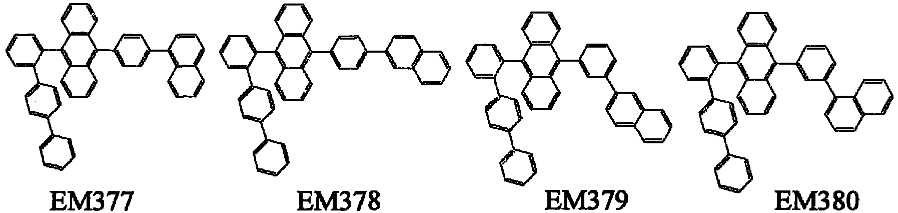

Specific examples of the fused aromatic amine derivative shown by the formula (2) are shown below.

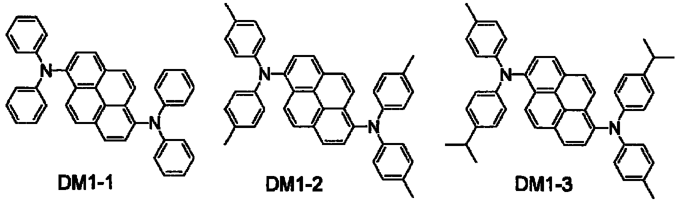

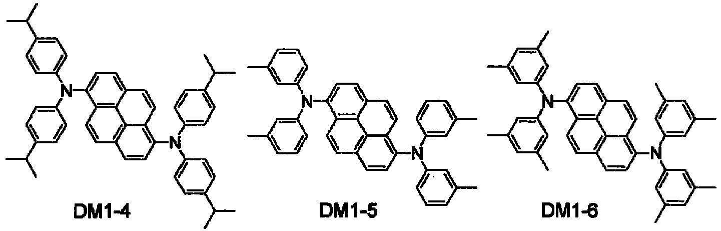

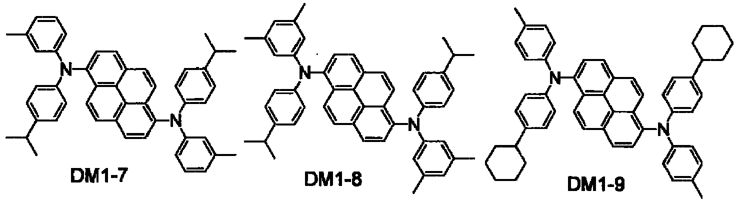

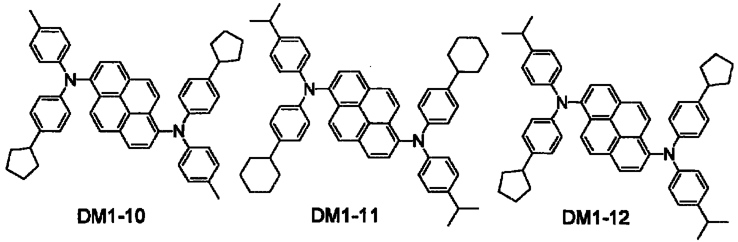

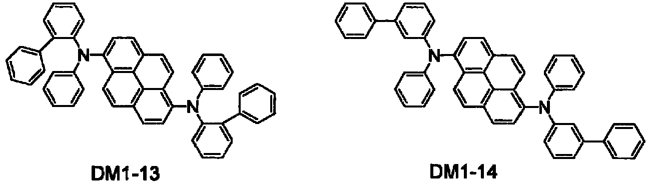

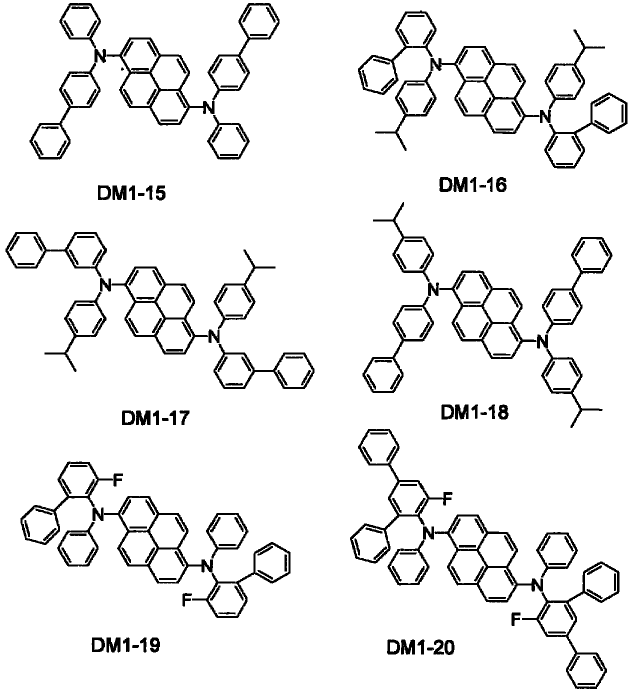

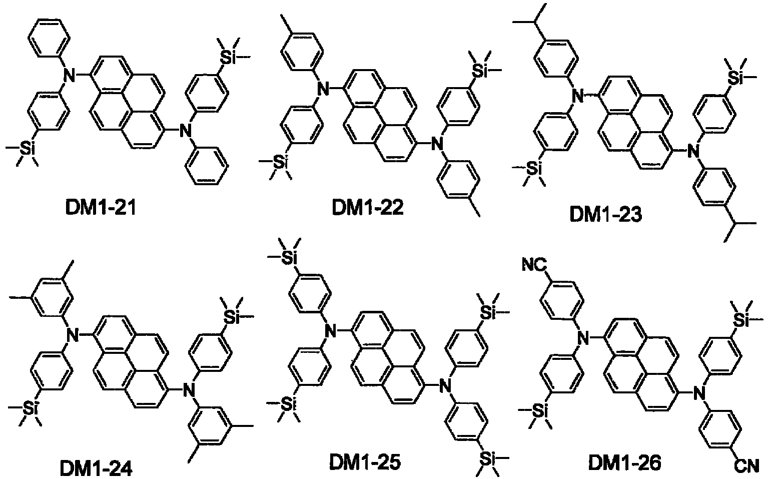

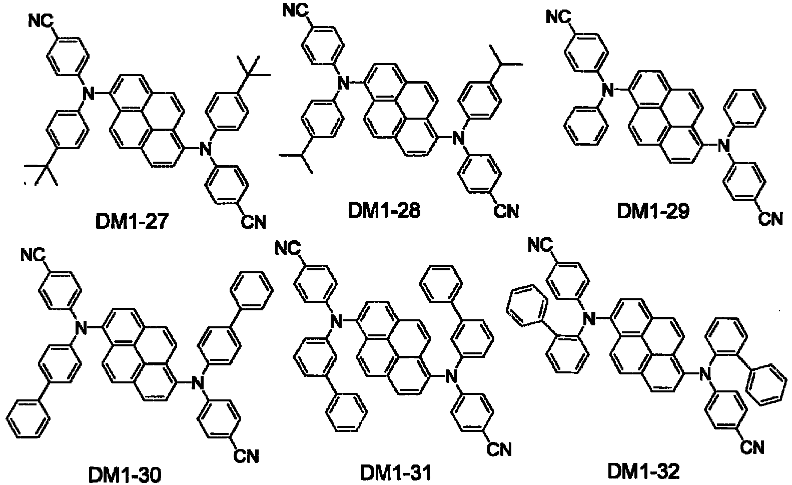

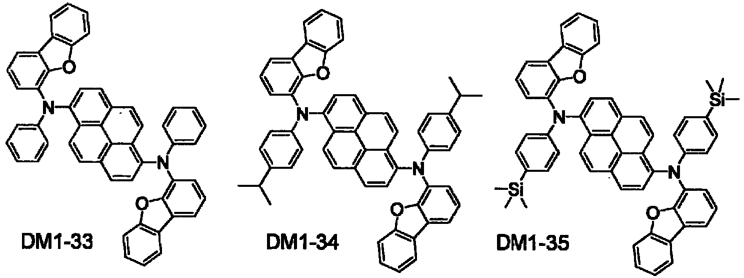

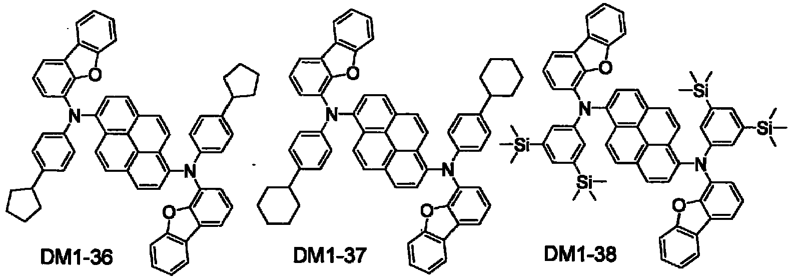

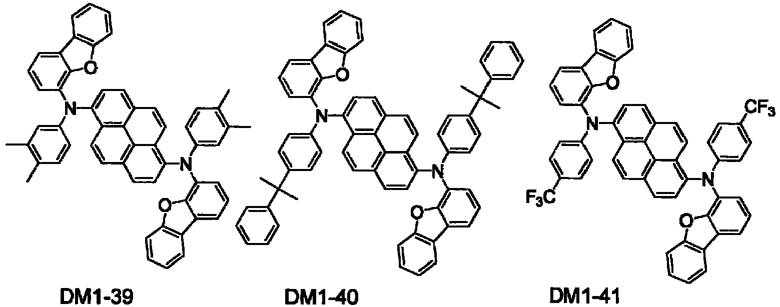

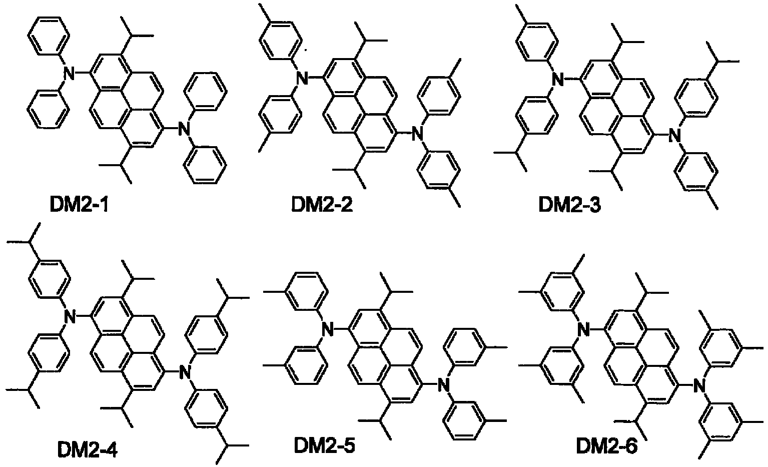

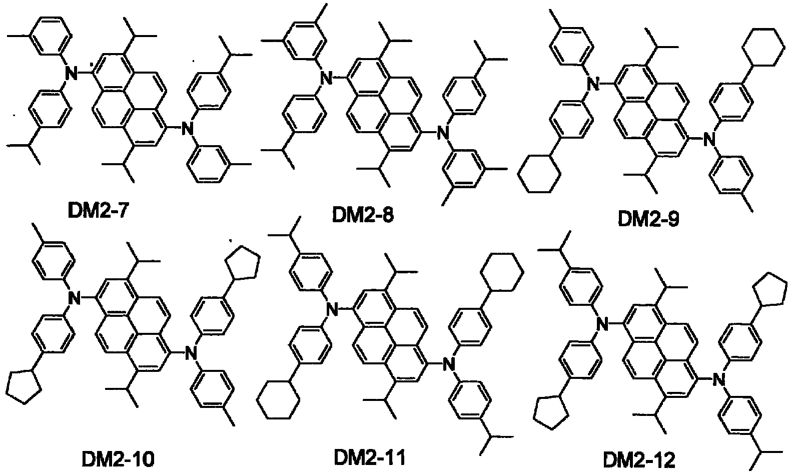

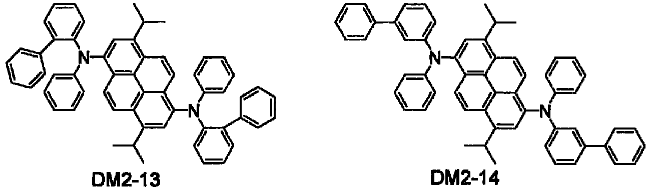

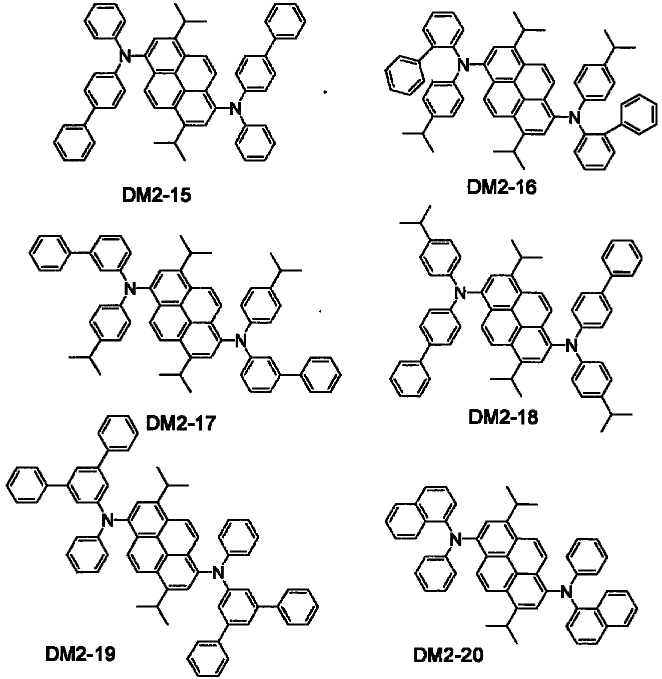

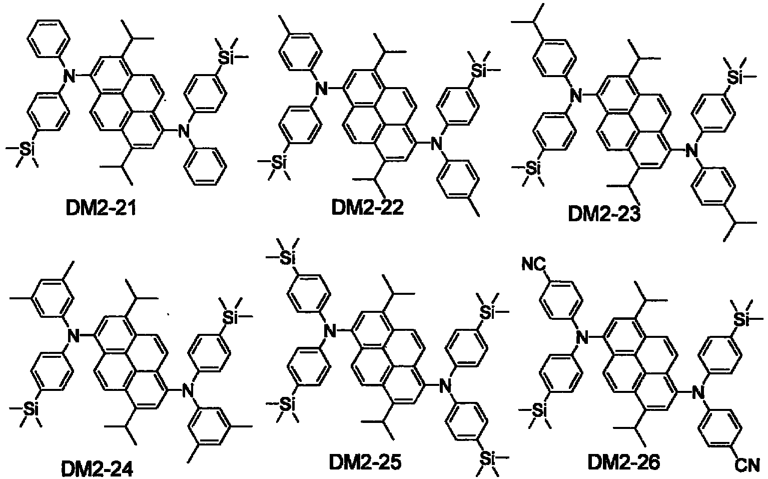

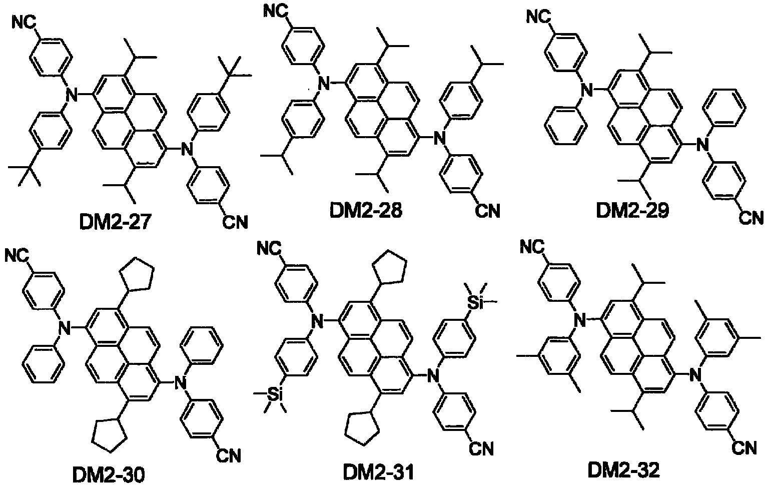

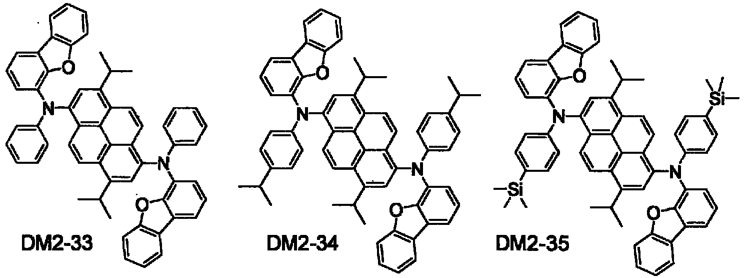

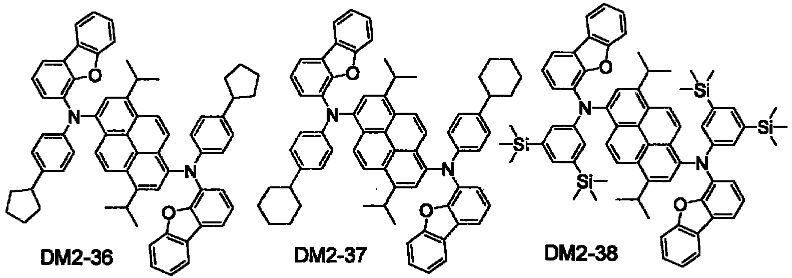

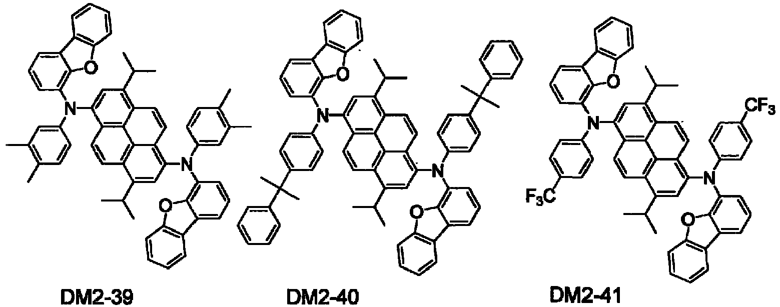

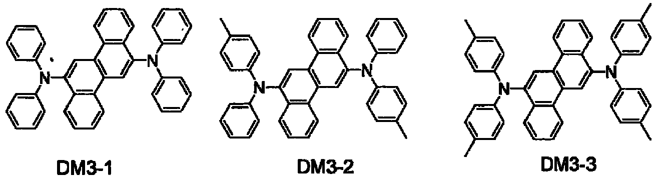

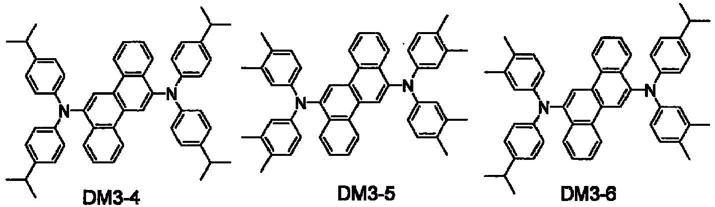

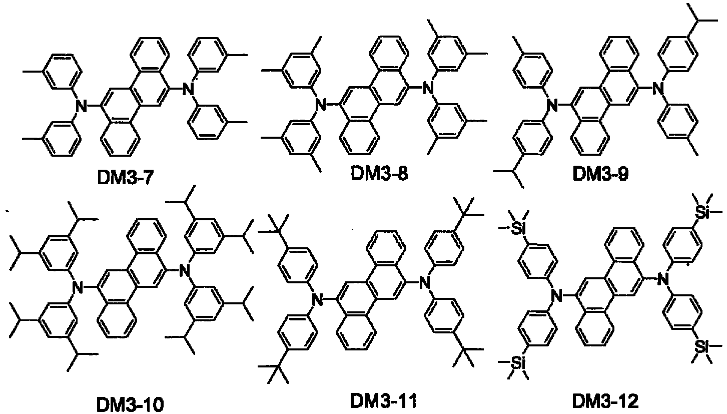

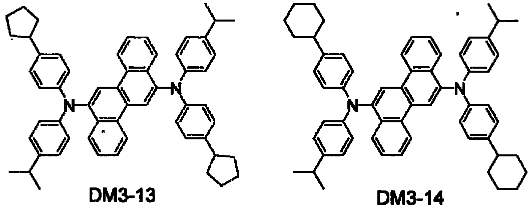

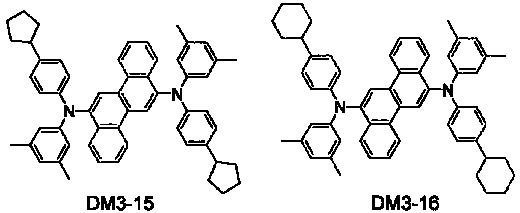

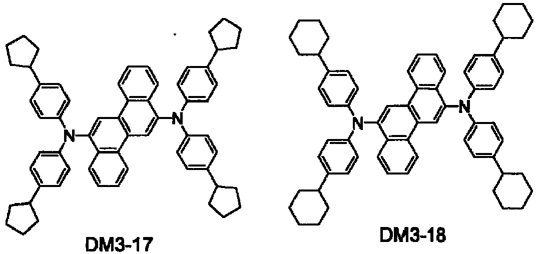

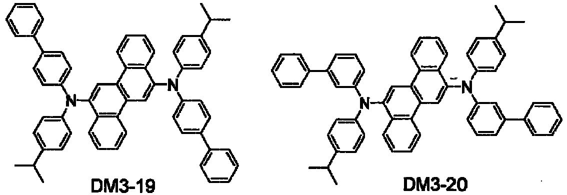

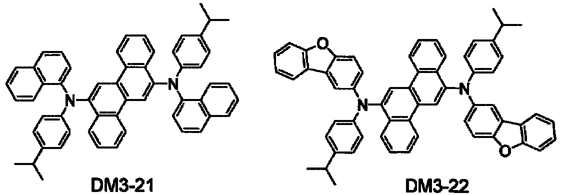

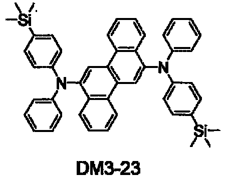

### Additional layer

The layer configuration of the organic EL device according to the invention is not particularly limited as long as the anode, the first organic layer, the second organic layer, and the cathode are sequentially stacked. The organic EL device may further include a plurality of additional organic layers.
The organic EL device according to the invention may have the following layer configurations (first to third embodiments), for example.

### <First embodiment>

The organic EL device according to the invention may have a layer configuration that includes at least one emitting layer, for example. Specific examples of the layer configuration are shown below.
(1) Anode/emitting layer/electron-injecting/transporting layer/cathode
(2) Anode/hote-injecting layer/emitting layer/electron-injecting/transporting layer/cathode
(3) Anode/hole-injecting layer/hole-transporting layer/emitting layer/electron-injecting/transporting layer/cathode
Among these, the configuration (3) may preferably be used. However, it is not limited to these configurations.

### <Second embodiment>

The organic EL device according to the invention may have a tandem device configuration that includes at least two emitting layers (units including an emitting layer), for example. The tandem device configuration normally includes a first emitting unit, a second emitting unit, and a carrier-generating layer (CGL) that is provided between the first emitting unit and the second emitting unit. The emitting unit sequentially includes at least a hole-transporting layer, an emitting layer, and an electron-transporting layer. The first organic layer according to the invention corresponds to the emitting layer, and the second organic layer according to the invention corresponds to the electron-transporting layer.
The emitting layer included in each emitting unit may be formed of a single layer, or may include a plurality of emitting layers.
The first organic layer according to the invention may be included in the first emitting unit, or may be included in the second emitting unit.

Specific examples of the tandem device configuration are shown below. Note that the emitting layer may be a laminate of a plurality of emitting layers.
Anode/fluorescent emitting layer/electron-injecting/transporting layer/carrier-generating layer/fluorescent emitting layer/cathode
Anode/fluorescent emitting layer/electron-injecting/transporting layer/carrier-generating layer/fluorescent emitting layer/barrier layer/cathode
Anode/phosphorescent emitting layer/carrier-generating layer/fluorescent emitting layer/electron-injecting/transporting layer/cathode
Anode/fluorescent emitting layer/electron-injecting/transporting layer/carrier-generating layer/phosphorescent emitting layer/cathode

### <Third embodiment>

The organic EL device according to the invention may sequentially include an anode, a plurality of emitting layers, an electron-injecting/transporting layer, and a cathode, and may further include a carrier barrier layer between two emitting layers among the plurality of emitting layers, the emitting layer that is adjacent to the carrier barrier layer being a fluorescent emitting layer, the fluorescent emitting layer corresponding to the first organic layer, and the electron-injecting/transporting layer corresponding to the second organic layer.

Examples of a preferable configuration of the organic EL device according to the third embodiment include a configuration in which an anode, a first emitting layer, a carrier barrier layer, a second emitting layer, and a cathode are sequentially stacked (see Japanese Patent No. 4134280, US2007/0273270A1, and WO2008/023623A1), wherein an electron-transporting region that includes a barrier layer that prevents diffusion of triplet excitons is provided between the second emitting layer and the cathode. The term "carrier barrier layer" used herein refers to a layer that adjusts injection of carriers into the emitting layer, and adjusts the carrier balance between electrons and holes injected into the emitting layer by providing an HOMO level/LUMO level energy barrier between the adjacent emitting layers.

Specific examples of the above configuration are shown below.
Anode/first emitting layer/charge barrier layer/second emitting layer/electron-injecting/transporting layer/cathode
Anode/first emitting layer/carrier barrier layer/second emitting layer/third emitting layer/electron-injecting/transporting layer/cathode
It is preferable to provide a hole-transporting region between the anode and the first emitting layer in the same manner as in the first and second embodiments.

The organic EL device according to the invention is preferably configured so that the first organic layer is adjacent to the second organic layer.

The substrate, the anode, the cathode, the hole-injecting layer, the hole-transporting layer, and the like included in the organic EL device according to the invention may be appropriately formed using the materials disclosed in WO2009/107596A1, WO2009/081857A1, US2009/0243473A1, US2008/0014464A1, US2009/0021160A1, and the like.

### EXAMPLES

### Example 1

The following materials were used to fabricate the organic EL device.

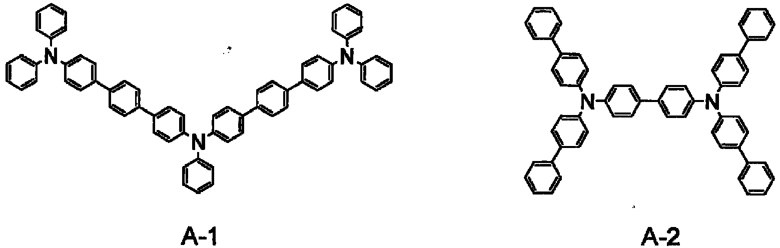

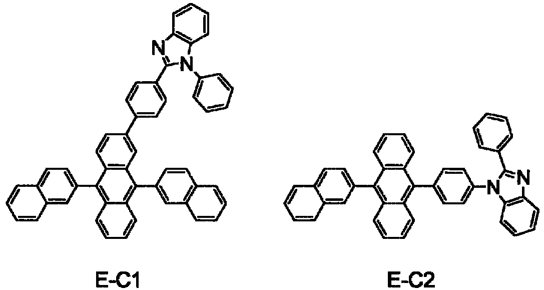

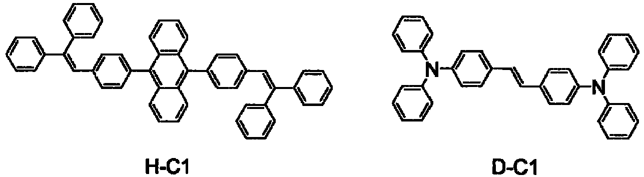

A glass substrate (manufactured by Geomatics) provided with an ITO transparent electrode (anode) (25×75×1.1 mm) was subjected to ultrasonic cleaning for 5 minutes in isopropyl alcohol, and then subjected to UV ozone cleaning for 30 minutes. The glass substrate was mounted on the substrate holder of a vacuum deposition apparatus, and the compound A-1 was deposited to a thickness of 50 nm on the side of the glass substrate on which the linear transparent electrode was formed so that the transparent electrode was covered with the compound A-1. The film of the compound A-1 functions as a hole-injecting layer. The compound A-2 was deposited on the film of the compound A-1 to form a film of the compound A-2 having a thickness of 45 nm. The film of the compound A-2 functions as a hole-transporting layer.
The compound EM2 (host material) and the compound DM2-34 (dopant material) were deposited on the film of the compound A-2 in a thickness ratio of 20:1 to obtain a blue-emitting layer (first organic layer) having a thickness of 25 nm. The compound ET2-5 (electron-transporting material) was deposited on the blue-emitting layer to form an electron-transporting layer (second organic layer) having a thickness of 25 nm. LiF was deposited on the electron-transporting layer to a thickness of 1 nm. Al was deposited on the LiF film to a thickness of 150 nm to form a metal cathode. An organic EL device was thus fabricated.

The device performance (drive voltage and emission wavelength) at a current density of 10 mA/cm² and the half life at an initial luminance of 1000 cd/cm² were measured using the organic EL device. The results are shown in Table 1.

### Examples 2 to 33 and Comparative Examples 1 to 5

An organic EL device was fabricated and evaluated in the same manner as in Example 1, except that the host material and the dopant material for the emitting layer (first organic layer) and the electron-transporting material for the electron-transporting layer (second organic layer) were changed as shown in Tables 1 and 2. The results are shown in Tables 1 and 2.

**TABLE 1**

| | Host material | Dopant material | Electron-transporting material | Drive voltage [V] | Emission wavelength [nm] | Half life [hour] |
|---|---|---|---|---|---|---|
| Example 1 | EM2 | DM2-34 | ET2-5 | 4.1 | 467 | 1500 |
| Example 2 | EM9 | DM2-34 | ET2-5 | 4.1 | 466 | 1500 |
| Example 3 | EM13 | DM2-34 | ET2-5 | 4.1 | 466 | 1600 |
| Example 4 | EM28 | DM2-34 | ET2-5 | 4.1 | 466 | 1700 |
| Example 5 | EM29 | DM2-34 | ET2-5 | 4.1 | 466 | 1700 |
| Example 6 | EM31 | DM2-34 | ET2-5 | 4.1 | 466 | 1700 |
| Example 7 | EM32 | DM2-34 | ET2-5 | 4.1 | 466 | 1700 |
| Example 8 | EM69 | DM2-34 | ET2-5 | 4.0 | 466 | 1700 |
| Example 9 | EM70 | DM2-34 | ET2-5 | 4.0 | 466 | 1700 |
| Example 10 | EM73 | DM2-34 | ET2-5 | 4.0 | 466 | 1700 |
| Example 11 | EM74 | DM2-34 | ET2-5 | 4.0 | 466 | 1500 |
| Example 12 | EM116 | DM2-34 | ET2-5 | 4.1 | 466 | 1600 |
| Example 13 | EM133 | DM2-34 | ET2-5 | 4.1 | 466 | 1600 |
| Example 14 | EM205 | DM2-34 | ET2-5 | 4.1 | 466 | 1500 |
| Example 15 | EM360 | DM2-34 | ET2-5 | 4.1 | 466 | 1500 |
| Example 16 | EM364 | DM2-34 | ET2-5 | 4.1 | 467 | 1500 |
| Example 17 | EM367 | DM2-34 | ET2-5 | 4.1 | 466 | 1500 |
| Example 18 | EM32 | DM1-6 | ET2-8 | 3.9 | 472 | 1400 |
| Example 19 | EM32 | DM1-20 | ET2-8 | 3.9 | 460 | 1300 |
| Example 20 | EM32 | DM1-25 | ET2-8 | 3.8 | 466 | 1300 |

**TABLE 2**

| | Host material | Dopant material | Electron-transporting material | Drive voltage [V] | Emission wavelength [nm] | Half life [hour] |
|---|---|---|---|---|---|---|
| Example 21 | EM32 | DM1-26 | ET2-8 | 3.6 | 458 | 1400 |
| Example 22 | EM32 | DM2-6 | ET2-8 | 3.9 | 473 | 1500 |
| Example 23 | EM32 | DM2-26 | ET2-8 | 3.6 | 460 | 1500 |
| Example 24 | EM32 | DM2-33 | ET2-8 | 3.9 | 461 | 1500 |
| Example 25 | EM32 | DM3-5 | ET2-8 | 3.9 | 467 | 1400 |
| Example 26 | EM32 | DM3-8 | ET2-8 | 3.9 | 459 | 1400 |
| Example 27 | EM31 | DM2-6 | ET1-1 | 4.2 | 473 | 1400 |
| Example 28 | EM31 | DM2-6 | ET2-14 | 3.6 | 473 | 1500 |
| Example 29 | EM31 | DM2-6 | ET2-17 | 3.6 | 473 | 1400 |
| Example 30 | EM31 | DM2-6 | ET2-18 | 4.1 | 473 | 1300 |
| Example 31 | EM31 | DM2-6 | ET2-19 | 4.0 | 473 | 1300 |
| Example 32 | EM31 | DM2-6 | ET2-21 | 4.1 | 473 | 1500 |
| Example 33 | EM31 | DM2-6 | ET2-30 | 3.7 | 473 | 1400 |
| Com. Ex. 1 | EM70 | DM2-34 | Alq | 6.5 | 466 | 1300 |
| Com. Ex. 2 | EM70 | DM2-34 | E-C1 | 5.5 | 466 | 1100 |
| Com. Ex. 3 | EM70 | DM2-34 | E-C2 | 4.1 | 466 | 700 |
| Com. Ex. 4 | H-C1 | DM2-34 | ET2-5 | 4.1 | 468 | 400 |
| Com. Ex. 5 | EM70 | D-C1 | ET2-5 | 4.1 | 440 | 300 |

The ionization potential (Ip), the energy gap (Eg), and the electron affinity (Ea) of the compounds used to fabricate the organic EL device are shown below.
The ionization potential (Ip) was measured using a thin film sample utilizing a system "AC-3" (manufactured by Riken Keiki Co., Ltd.). The energy gap (Eg) was calculated from the absorption edge of the absorption spectrum in a toluene solution of the compound. The electron affinity (Ea) was calculated by the expression "Ea=Ip-Eg".

**TABLE 3**

| | EM70 | DM2-34 | ET2-5 | EC-1 | EC-2 | A-2 |
|---|---|---|---|---|---|---|
| Ip [eV] | 6.0 | 5.5 | 6.0 | 6.0 | 6.0 | 5.5 |
| Eg [eV] | 3.0 | 2.8 | 2.9 | 2.9 | 3.0 | 3.1 |
| Ea [eV] | 3.0 | 2.7 | 3.1 | 3.1 | 3.0 | 2.4 |

Since the aminochrysene derivative or the aminopyrene derivative (dopant material) that is the fused aromatic amine derivative shown by the formula (2) has an ionization potential lower than that of the host material that is the monoanthracene derivative shown by the formula (1), the aminochrysene derivative or the aminopyrene derivative traps holes. Therefore, the organic EL device fabricated using the aminochrysene derivative or the aminopyrene derivative show a tendency that holes remain around the interface of the emitting layer with the hole-transporting layer.

Since the difference in affinity (Af) between the electron-transporting material EC-2 and the host material is small, electrons are smoothly injected into the emitting layer. Moreover, since the number of electron traps in the emitting layer is small, electrons easily reach the interface (boundary) between the emitting layer and the hole-transporting layer. Since the hole-transporting material and the host material differ in affinity (Af), electrons are blocked at the interface between the hole-transporting layer and the emitting layer. However, electrons partially enter the hole-transporting layer, and cause the hole-transporting material to deteriorate. This results in a decrease in lifetime.

Since the monoanthracene derivative shown by the formula (1) (host material) and the heterocyclic derivative shown by the formula (3) (electron-transporting material) according to the invention differ in affinity (Af) by about 0.1 eV, a part of electrons accumulate at the interface between the emitting layer and the electron-transporting layer. This causes holes that accumulate at the interface between the hole-transporting layer and the emitting layer to be introduced into the emitting layer, so that a deterioration in the hole-transporting material is suppressed.
An increase in drive voltage normally occurs when the emitting layer and the electron-transporting layer differ in affinity (Af). However, the lifetime can be improved without causing an increase in drive voltage by introducing a specific heterocyclic substituent into the electron-transporting material according to the invention.

As described above, the relationship between the affinity (Af) of the host material that utilizes an appropriate dopant and the affinity (Af) of the electron-transporting material that has an appropriate partial structure is important. It is preferable that the host material have an affinity (Af) of 3.0 eV or less. Since an anthracene derivative that includes a styryl group (e.g., H-C1) or a bisanthracene derivative has an affinity (Af) of about 3.1 eV, the emitting layer and the electron-transporting layer do not differ in affinity (Af). Therefore, the lifetime is not improved. The drive voltage could not be reduced in Comparative Example 2 although the emitting layer and the electron-transporting layer differed in affinity (Af).

As is clear from the results for Examples, it is considered that a decrease in voltage and an increase in lifetime can both be implemented by utilizing a combination of the monoanthracene derivative shown by the formula (1) (host material), an aminochrysene derivative or an aminopyrene derivative (dopant material), and the heterocyclic derivative shown by the formula (3) that is substituted with a specific heterocyclic ring (electron-transporting material).

### INDUSTRIAL APPLICABILITY

The organic EL device according to the invention may be used for a large television display panel, an illumination panel, and the like for which a reduction in power consumption and an increase in lifetime are desired.

Although only some exemplary embodiments and/or examples of the invention have been described in detail above, those skilled in the art will readily appreciate that many modifications are possible in the exemplary embodiments and/or examples without materially departing from the novel teachings and advantages of the invention. Accordingly, all such modifications are intended to be included within the scope of the invention.
The documents described in the specification are incorporated herein by reference in their entirety.

## Claims

1. An organic electroluminescence device in order comprising an anode, a first organic layer, a second organic layer, and a cathode, the first organic layer comprising a monoanthracene derivative shown by a formula (1) and a fused aromatic amine derivative shown by a formula (2), and the second organic layer comprising a heterocyclic derivative shown by a formula (3), 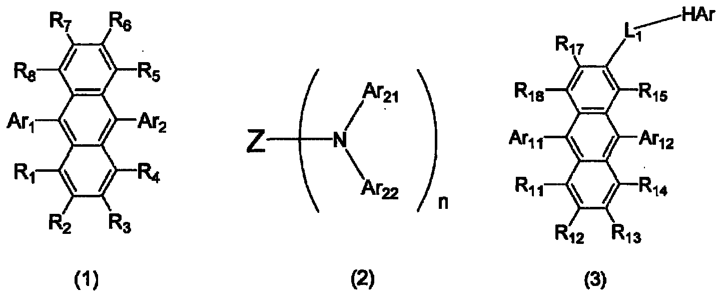 wherein R₁ to R₈, R₁₁ to R₁₅, R₁₇, and R₁₈ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, Ar₁, Ar₂, Ar₁₁, Ar₁₂, Ar₂₁, and Ar₂₂ are independently a substituted or unsubstituted aromatic hydrocarbon ring group having 6 to 30 ring carbon atoms, or a substituted or unsubstituted heterocyclic group having 5 to 30 ring atoms, provided that the substituted aromatic hydrocarbon ring group or the substituted heterocyclic group represented by Ar₁ and Ar₂ is substituted with a substituent other than a styryl group, Z is a substituted or unsubstituted chrysene residue or a substituted or unsubstituted pyrene residue, n is an integer from 1 to 4, L₁ is a substituted or unsubstituted divalent aromatic hydrocarbon ring group having 6 to 30 ring carbon atoms or a substituted or unsubstituted divalent heterocyclic group having 5 to 30 ring atoms, and HAr is any of nitrogen-containing heterocyclic groups shown by formulas (4) to (6), 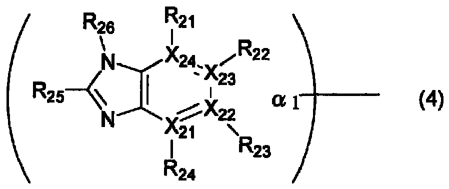 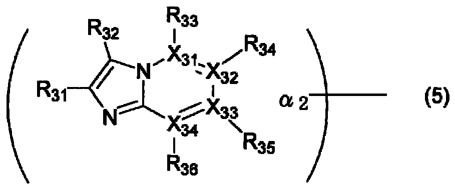 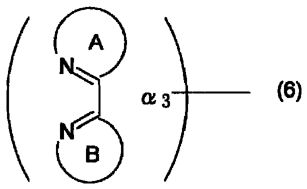 wherein R₂₁ to R₂₆ and R₃₁ to R₃₅ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituents among R₂₁ to R₂₄ and R₃₁ to R₃₆ may form a saturated or unsaturated ring, X₂₁ to X₂₄ and X₃₁ to X₃₄ are independently a carbon atom or a nitrogen atom, provided that R₂₁ is absent when X₂₄ is a nitrogen atom, R₂₂ is absent when X₂₃ is a nitrogen atom, R₂₃ is absent when X₂₂ is a nitrogen atom, R₂₄ is absent when X₂₁ is a nitrogen atom, R₃₃ is absent when X₃₁ is a nitrogen atom, R₃₄ is absent when X₃₂ is a nitrogen atom, R₃₅ is absent when X₃₃ is a nitrogen atom, and R₃₆ is absent when X₃₄ is a nitrogen atom, A and B are independently a substituted or unsubstituted 5-membered ring or a substituted or unsubstituted 6-membered ring, provided that adjacent substituens that substitute the 5-membered ring or the 6-membered ring represented by A and B may form a saturated or unsaturated ring, α₁ is bonded to R₂₁, R₂₂, R₂₃, R₂₄, or R₂₆, α₂ is bonded to one of R₃₁ to R₃₆, and α₃ is bonded to the nitrogen-containing ring represented by A or the nitrogen-containing ring represented by B, provided that R₂₁, R₂₂, R₂₃, R₂₄, or R₂₆ that is bonded to α₁ is a single bond, and one of R₃₁ to P₃₆ that is bonded to α₂ is a single bond.

2. The organic electroluminescence device according to claim 1, wherein HAr is any of heterocyclic groups shown by formulas (7) to (11), 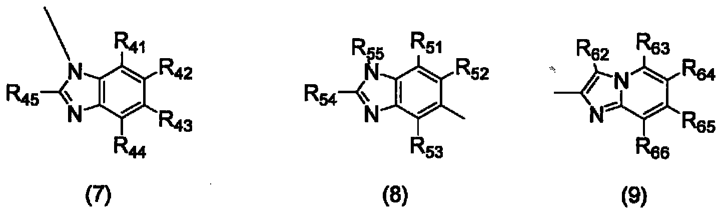 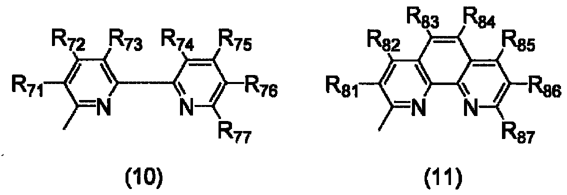 wherein R₄₁ to R₄₅, R₅₁ to R₅₅, R₆₂ to R₆₆, R₇₁ to R₇₇, and R₈₁ to R₈₇ are independently a hydrogen atom, a halogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aryloxy group having 6 to 30 ring carbon atoms, a substituted or unsubstituted aromatic hydrocarbon ring group having 6 to 30 ring carbon atoms, or a substituted or unsubstituted heterocyclic group having 5 to 30 ring atoms, provided that adjacent substituents among R₄₁ to R₄₄, R₅₁, R₅₂, R₆₂ to R₆₆, R₇₁ to R₇₇, and R₈₁, to R₈₇ may bond to each other to form a saturated or unsaturated ring.

3. The organic electroluminescence device according to claim 1 or 2, further comprising a layer between the second organic layer and the cathode, the layer being formed of one or more substances selected from the group consisting of alkali metal oxides, alkali metal halides, alkaline-earth metal oxides, alkaline-earth metal halides, rare earth metal oxides, rare earth metal halides, organic complexes of alkali metals, organic complexes of alkaline-earth metals, and organic complexes of rare earth metals.

4. The organic electroluminescence device according to any one of claims 1 to 3, wherein the fused aromatic amine derivative shown by the formula (2) is a compound shown by a formula (12), 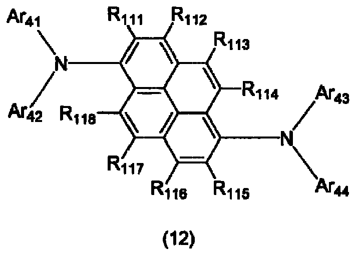 wherein R₁₁₁ to R₁₁₈ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₄₁ to Ar₄₄ are independently a substituted or unsubstituted aromatic hydrocarbon ring group having 6 to 30 ring carbon atoms or a substituted or unsubstituted heterocyclic group having 5 to 30 ring atoms.

5. The organic electroluminescence device according to claim 4, wherein at least one of R₁₁₂ and R₁₁₆ in the formula (12) is a substituted or unsubstituted alkyl group having 1 to 10 carbon atoms or a substituted or unsubstituted cycloalkyl group having 3 to 10 carbon atoms.

6. The organic electroluminescence device according to any one of claims 1 to 3, wherein the fused aromatic amine derivative shown by the formula (2) is a compound shown by a formula (13), 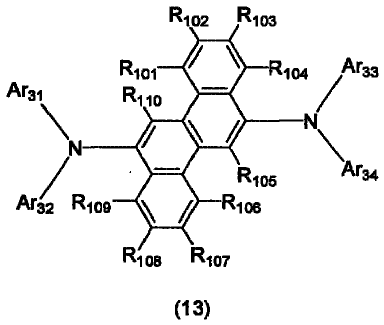 wherein R₁₀₁ to R₁₁₀ are independently a hydrogen atom, a halogen atom, a substituted or unsubstituted alkyl group having 1 to 10 carbon atoms, a substituted or unsubstituted cycloalkyl group having 3 to 10 ring carbon atoms, a substituted or unsubstituted alkylsilyl group having 3 to 20 carbon atoms, a substituted or unsubstituted arylsilyl group having 6 to 30 ring carbon atoms, a substituted or unsubstituted alkoxy group having 1 to 10 carbon atoms, a substituted or unsubstituted aralkyl group having 6 to 20 ring carbon atoms, a substituted or unsubstituted aryloxy group having 6 to 30 ring carbon atoms, or a cyano group, and Ar₃₁, to Ar₃₄ are independently a substituted or unsubstituted aromatic hydrocarbon ring group having 6 to 30 ring carbon atoms or a substituted or unsubstituted heterocyclic group having 5 to 30 ring atoms.

7. The organic electroluminescence device according to any one of claims 1 to 6, wherein the first organic layer is in contact with the second organic layer.
